(19) Europäisches Patentamt
European Patent Office
Office européen des brevets

(11) **EP 3 690 063 B1**

(12) **EUROPEAN PATENT SPECIFICATION**

(45) Date of publication and mention
of the grant of the patent:
**20.08.2025 Bulletin 2025/34**

(21) Application number: **19217908.3**

(22) Date of filing: **24.11.2015**

(51) International Patent Classification (IPC):
**C12Q 1/6886** (2018.01)

(52) Cooperative Patent Classification (CPC):
**C12Q 1/6886;** C12Q 2600/154

(54) **DIAGNOSIS OF LUNG CANCER**

DIAGNOSE VON LUNGENKREBS

DIAGNOSTIC DU CANCER DU POUMON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.11.2014 EP 14194732**

(43) Date of publication of application:
**05.08.2020 Bulletin 2020/32**

(62) Document number(s) of the earlier application(s) in accordance with Art. 76 EPC:
**15801743.4 / 3 224 377**

(73) Proprietor: **AIT Austrian Institute of Technology GmbH**
**1210 Wien (AT)**

(72) Inventors:
• **WIELSCHER, Matthias**
**1180 Wien (AT)**
• **WEINHÄUSEL, Andreas**
**7311 Neckenmarkt (AT)**
• **VIERLINGER, Klemens**
**1090 Wien (AT)**
• **NOEHAMMER, Christa**
**3420 Kritzendorf (AT)**
• **ZIESCHE, Rolf**
**1210 Wien (AT)**
• **GSUR, Andrea**
**1130 Wien (AT)**

(74) Representative: **Vossius & Partner**
**Patentanwälte Rechtsanwälte mbB**
**Siebertstrasse 3**
**81675 München (DE)**

(56) References cited:
**EP-A1- 2 253 714**

• X. XUE ET AL: "Circulating DNA and Lung Cancer", ANNALS OF THE NEW YORK ACADEMY OF SCIENCES, vol. 1075, no. 1, 1 September 2006 (2006-09-01), pages 154 - 164, XP055191925, ISSN: 0077-8923, DOI: 10.1196/ annals.1368.021
• BREMNES R M ET AL: "Circulating tumour-derived DNA and RNA markers in blood: a tool for early detection, diagnostics, and follow-up?", LUNG CANCER, ELSEVIER, AMSTERDAM, NL, vol. 49, no. 1, 1 July 2005 (2005-07-01), pages 1 - 12, XP027774224, ISSN: 0169-5002, [retrieved on 20050701]
• RAUCH TIBOR A ET AL: "DNA methylation biomarkers for lung cancer", TUMOR BIOLOGY, KARGER, BASEL, CH, vol. 33, no. 2, Sp. Iss. SI, 1 April 2012 (2012-04-01), pages 287 - 296, XP009502625, ISSN: 1010-4283, [retrieved on 20111206], DOI: 10.1007/S13277-011-0282-2
• S KALARI ET AL: "The DNA methylation landscape of small cell lung cancer suggests a differentiation defect of neuroendocrine cells", ONCOGENE, vol. 32, no. 30, 1 July 2013 (2013-07-01), London, pages 3559 - 3568, XP055707049, ISSN: 0950-9232, DOI: 10.1038/ onc.2012.362
• REJANE HUGHES CARVALHO ET AL: "Genome-wide DNA methylation profiling of non-small cell lung carcinomas", EPIGENETICS & CHROMATIN, BIOMED CENTRAL LTD, LONDON, UK, vol. 5, no. 1, 22 June 2012 (2012-06-22), pages 9, XP021127600, ISSN: 1756-8935, DOI: 10.1186/ 1756-8935-5-9

EP 3 690 063 B1

- **CAIYUN G. LI ET AL: "PAX Genes in Cancer; Friends or Foes?", FRONTIERS IN GENETICS, vol. 3, 1 January 2012 (2012-01-01), Switzerland, XP055707053, ISSN: 1664-8021, DOI: 10.3389/ fgene.2012.00006**

Remarks:
The complete document including Reference Table(s) and the Sequence Listing(s) can be downloaded from the EPO website

Remarks:

**Description**

The present invention is defined by the claims. The present disclosure generally relates to specifically methylated nucleic acids, methods of diagnosing lung cancer or a predisposition therefor, and a corresponding kit.

Cell-free DNA methylation analysis from blood is useful as minimally invasive diagnostic tool for early detection or disease monitoring. It reduces the need for biopsies or sophisticated imaging methods, which, in case of the lung, can introduce sampling artefacts due to the large area and the complex branching structure of the lung (Alberg et al., Chest 143, e1S-29S (2013)). On the other hand, biopsies were and are established and useful means for early detection or disease monitoring.

Lung diseases are very common. Various studies report a lifetime risk for lung cancer of 6-8% (Fauci, Harrison's principles of internal medicine, McGraw-Hill Medical New York (2008)), which is leading the number of cancer associated deaths worldwide (Alberg (2013), loc. cit.). Evidence derived from minimal invasive tests may aid in early diagnosis and reduce death rates.

A major prerequisite for early diagnosis or monitoring of lung diseases are suitable molecular markers. In this regard, DNA methylation is highly tissue specific (Esteller, N Engl J Med 358, 1148-1159 (2008)). Furthermore, DNA methylation changes can be detected early in cancer development (Brock et al., N Engl J Med 358, 1118-1128 (2008)), which makes it more suitable for early detection than mutation analysis (Brock (2008), loc. cit., Shinozaki et al. Clinical cancer research, 13, 2068-2074 (2007)). For example in cancer, high frequencies of specific methylation alterations were observed already in the early onset of cancer development (Brock (2008), loc. cit.). This frequency is lower in mutation analysis of cancer-derived cell free DNA (cfDNA) (Brock (2008), loc. cit., Shinozaki (2007), loc. cit.).

Commonly used methods for DNA methylation analysis rely on bisulfite conversion of DNA, which allows detection of single CpGs.

Methylation of certain genes in a lung cancer context has been described in the art. Rauch et al. (Cancer Research 66, 7939-7947 (2006)) describe the application of a methylated-CpG island recovery assay (MIRA) to lung cancer cells. Especially homeodomain containing genes were discovered. Kalari et al. (Oncogene 32, 3559-3568 (2013)) characterize the DNA methylation landscape of small cell lung cancer. A further example is EP 2 253 714 which describes the methylation of the PCDHGA12 gene in lung cancer cells.

US2010/0273151 describes a large number of genes which are differentially methylated when comparing medulloblastoma samples with normal cerebellum. Similarly, US2011/0318738 describes a host of genes which are differentially methylated in FAP adenoma tissue. The medical indication which is lung cancer is not mentioned in these documents.

The present inventors discovered new markers as well as previously unknown disease-relevant methylation positions in markers which, as such, were previously described. Moreover, not only differentially methylated genes and positions are provided, but also methods of diagnosing and disease monitoring.

In view of the prior art, the technical problem underlying the present invention can be seen in the provision of alternative or improved means and methods for diagnosing lung cancer.

The technical problem has been solved by the subject-matter of the claims enclosed herewith. Accordingly, in a first aspect, the present invention relates to a nucleic acid comprising or consisting of

(a) the sequence
(4) from position 4 to position 84 of SEQ ID NO: 1, wherein the cytidine in at least one CpG dinucleotide comprised in said sequence is methylated;
or
(b) a sequence heaving at least 80% sequence identity to a sequence of (a).

The term "nucleic acid" has its art-established meaning. It relates to a polycondensate of nucleotides. The nucleic acid is preferably DNA. Accordingly, the group of nucleotide building blocks comprises or consists of adenine, guanine, cytosine and thymidine, commonly abbreviated as A, G, C and T.

While not being preferred, it is envisaged that one, more or all nucleotides in any nucleic acid (including probes and primers) as described herein may comprise one or more art-established modifications. Art-established modifications include modifications of the 2'-position of the sugar moiety which is desoxyribose in case of DNA. An example of a 2'-modification is 2'-O-methyl. Another art-established modification of the sugar phosphate backbone is the replacement of oxygen with sulfur in a phosphate moiety. Correspondingly modified phosphates are known, for example, as phosphorothioate.

Further included are nucleic acid mimicking molecules known in the art such as synthetic or semisynthetic derivatives of DNA or RNA and mixed polymers, to be used as sense and/or antisense strands. They may contain additional non-natural or derivatized nucleotide bases, as will be readily appreciated by those skilled in the art. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA), locked nucleic acid (LNA) (see, for example, Braasch and Corey, Chemistry & Biology 8, 1-7 (2001)) and peptide nucleic acid (PNA).

LNA is an RNA derivative in which the ribose ring is constrained by a methylene linkage between the 2'-oxygen and the 4'-carbon.

For the purposes of the present invention, a peptide nucleic acid (PNA) is a polyamide type of DNA analog. The monomeric units for the corresponding derivatives of adenine, guanine, thymine and cytosine are available commercially (for example from Perceptive Biosystems). PNA is a synthetic DNA-mimic with an amide backbone in place of the sugar-phosphate backbone of DNA or RNA. As a consequence, certain components of DNA, such as phosphorus, phosphorus oxides, or deoxyribose derivatives, are not present in PNAs. As disclosed by Nielsen et al., Science 254:1497 (1991); and Egholm et al., Nature 365:666 (1993), PNAs bind specifically and tightly to complementary DNA strands and are not degraded by nucleases. Furthermore, they are stable under acidic conditions and resistant to proteases (Demidov et al. (1994), Biochem. Pharmacol., 48, 1310-1313). Their electrostatically neutral backbone increases the binding strength to complementary DNA as compared to the stability of the corresponding DNA-DNA duplex (Wittung et al. (1994), Nature 368, 561-563; Ray and Norden (2000), Faseb J., 14, 1041-1060). In fact, PNA binds more strongly to DNA than DNA itself does. This is probably because there is no electrostatic repulsion between the two strands, and also the polyamide backbone is more flexible. Because of this, PNA/DNA duplexes bind under a wider range of stringency conditions than DNA/DNA duplexes, making it easier to perform multiplex hybridization. Smaller probes can be used than with DNA due to the strong binding. In addition, it is more likely that single base mismatches can be determined with PNA/DNA hybridization because a single mismatch in a PNA/DNA 15-mer lowers the melting point ($T_m$) by 8°-20° C, vs. 4°-16° C for the DNA/DNA 15-mer duplex. Thereby discrimination between perfect matches and mismatches is improved. For its uncharged nature, PNA also permits the hybridisation of DNA samples at low salt or no-salt conditions, since no inter-strand repulsion as between two negatively charged DNA strands needs to be counteracted. As a consequence, the target DNA has fewer secondary structures under hybridisation conditions and is more accessible to probe molecules.

PNA chimera according to the present invention are molecules comprising one or more PNA portions. The remainder of the chimeric molecule may comprise one or more DNA portions (PNA-DNA chimera).

A further modification in accordance with the invention is the conversion of cytosine to uracil. When an accordingly modified nucleic acid is amplified by conventional techniques, uracil is converted into thymidine. The conversion of C into U can be performed experimentally by treatment with bisulfite. Alternatively, it can be performed *in silico* by corresponding software programs. Either method is well-established in the art. To the extent cytosine is methylated, it is not amenable to conversion. Accordingly, correspondingly modified sequences of the present invention are those where all Cs are converted into Ts, including those in CpG dinucleotides which in principle are amenable to methylation, but are not methylated under the circumstances given. Also provided are modified sequences where one, more or all of the Cs within CpG dinucleotides are methylated and accordingly not amenable to conversion from C to T. In the latter sequences, only those Cs are converted into Ts which are not methylated in the parent sequence. Preferred sequences in this regard are subject of a preferred embodiment detailed further below. Not only C/T converted sequences, but also C/U converted sequence are embraced by the invention.

Methylation of DNA typically occurs in regions where CpG dinucleotides are overrepresented as compared to their average occurrence within a given genome. For the purposes of the present invention, we refer to such regions where CpG dinucleotides occur more frequently as "CpG clusters". We note that there exists also the art-established term "CpG island". Generally, the term "CpG island" designates a stretch of up to 500 base pairs in length where more than 60% of the base sequence is present in the form of CpG dinucleotides. There may be flanking regions on one or both sides of a given CpG island where CpG dinucleotides are still overrepresented as compared to the genome average. Such flanking sequences are generally referred to as "CpG shores". The term "CpG cluster" as used herein differs from these art-established definitions in that there is no minimum requirement as regards the frequency of CpG dinucleotide motifs. A CpG cluster in accordance with the present invention is a sequence of typically 100 nucleotides or less which contains at least two CpG dinucleotide motifs which are amenable to methylation. While CpG islands according to the art-established definition have a length of up to 500 base pairs the CpG clusters disclosed above are of length below 100 base pairs, we note that regions with an elevated frequency of CpG dinucleotides may be have lengths of up to 1000 or even 3000 bases. The abbreviation "CpG" designates a dinucleotide motif occurring within a given DNA strand. Typically, methylated bases are cytosines comprised in such dinucleotide motifs. To give an example, the subsequence from position 4 to position 84 of SEQ ID NO: 1 comprises five CpG dinucleotide motifs. The subsequence of SEQ ID NO: 1 from position 4 to position 84 accordingly defines a CpG cluster, in this specific case a previously unknown CpG cluster in the genomic DNA of the PAX9 gene. Generally speaking, the first aspect defines in each of items (1) to (46) a disease-associated CpG cluster.

The enzymatically catalyzed process of methylation of cytosine yields 5-methyl cytosine. Methylated forms of DNA may be further derivatized within a cell to give rise to cytosines which bear a hydroxymethyl group in the 5 position. "Methylated cytosine" in accordance with the present invention is either 5-methyl cytosine or 5-hydroxymethyl cytosine, 5-methyl cytosine being preferred. The enzymes catalyzing methylation of DNA are known as DNA methyl transferases.

DNA modification by the process of methylation is a form of epigenetic gene regulation. DNA methylation as well as hydroxymethylation is frequently associated with development and/or disease.

If a position is methylated in accordance with the present invention, it is generally or preferably methylated to a high degree, for example more than 60%, more than 70%, more than 80%, more than 90%, more than 95%, more than 98%, more than 99% or complete; i.e. 100% methylation. As such, the methylation status of a particular position can generally be viewed as a Boolean variable: a given position is either unmethylated or methylated. The term "unmethylated" in accordance with the present invention refers to low degrees of methylation, preferably less than 20%, less than 10%, less than 5%, less than 2% or less than 1%. Also included within the term "unmethylated" is the complete absence of methylation or the absence of detectable methylation. Furthermore, it is generally observed that all cytosines of the CpG motifs of a given CpG cluster are either methylated or not. Accordingly, it is preferred that within a given CpG cluster, the degree of methylation does not vary among the individual CpG motifs by more than 40%, more than 30%, more than 20%, more than 10%, more than 5%, more than 2%, or more than 1%. In other words, preferably either are all positions within a given CpG cluster methylated or they are not.

The preferred percentages given above relate in particular to those cases where a sample is directly drawn from a tumor, for example by means of a biopsy. Having said that, we note that in accordance with the preferred embodiment detailed further below, the methods in accordance with the present invention are performed by using samples of cell-free DNA. While in patients suffering from lung cancer, (i) the amount of cell-free DNA is generally increased, and (ii) cell-free DNA originating from the cancer is present among the cell-free DNA comprised in serum, it will also be generally true that a significant amount of cell-free DNA which does not originate from the cancer is present in the sample. The cell-free DNA obtained from a lung cancer patient typically contains a mixture of methylated and non-methylated DNA. Accordingly, while the nucleic acid defined in accordance with the first aspect of the invention, when originating from a lung cancer, will generally be methylated, the corresponding sequences originating from other parts of the body of the patient will generally not be methylated.

Under such circumstances, the methods of diagnosing and disease monitoring in accordance with the present invention can nevertheless be practiced. A preferred measure of the methylation status is the fold change (F.C.). The fold change is the ratio of the amount of methylated DNA in a sample from a patient suffering from lung cancer over the amount of methylated DNA from an individual which does not suffer from lung cancer for a given gene, fragment thereof, CpG cluster or CpG dinucleotide, respectively. In addition, logarithms of the fold change may be given. The fold change data in the tables enclosed herewith are logarithms. The term "amount" includes molar concentration, mass per unit volume (w/v%), mass, number of molecules, and relative amount with respect to (a) reference gene(s). The terms "patient" and "individual" refer to single persons as well as averages of a cohort and population averages. A preferred sample is plasma. A preferred individual who does not suffer from lung cancer is a healthy and/or non-cancer-control individual. Preferred forms of lung cancer are given further below. Consistent with the use of fold changes for the purpose of diagnosis and disease monitoring, the respective CpG dinucleotide motifs and CpG clusters are also referred to as being differentially methylated. In case of the markers in accordance with the present invention, the diseased state, i.e. lung cancer, is characterized by a higher degree of methylation as compared to the healthy state, and, more generally any non-cancer state. Owing to the background of cell-free DNA originating from healthy tissue, though, the above mentioned high percentages of methylation, although typically observed in biopsies, will not be observed in cell-free DNA samples.

Having said that, the use of a control (in case of the fold change as defined above a sample from an individual which does not suffer from lung cancer) is not indispensable when analyzing any type of sample such as cell-free DNA. To explain further, the method of the present invention may be calibrated. Calibration may be for a given type of sample, a given workflow of sample processing, and/or a given gene, CpG cluster or CpG dinucleotide. Calibration results in a threshold value for a given measure of the methylation status. The threshold distinguishes diseased from non-diseased samples as well as methylated from non-methylated samples. For the sake of completeness we note that also in the scenario described above (background of non-methylated markers in cell-free DNA samples) a given CpG dinucleotide, CpG cluster or gene may be classified as being methylated, for example either based on fold change or the above mentioned calibration. In such context, the status "methylated" refers to a status where it is the contribution to the total amount of cell-free DNA which contribution originates from the tumor, metastasis and/or circulating tumor cells, and accounts for a component of methylated DNA. This component is detected and provides for a classification as being methylated based on fold change or comparison with the above mentioned threshold value.

The fold change is a generally applicable measure and is not confined to the analysis of cell-free DNA.

If not indicated otherwise, the term "methylation" as used in the following refers to the state of being methylated, rather than to the chemical reaction.

Nucleic acids in accordance as described herein may either consist of the specifically indicated subsequence of a given entry of the sequence listing (e.g. from position 4 to position 84 of SEQ ID NO: 1), or may comprise said subsequence. In the latter case, one or two flanking sequences may be present upstream, downstream or upstream and downstream to the specifically indicated subsequence. Flanking sequences preferably are in the range of 100, 200, 300, 400 or 500 base pairs. To the extent the first aspect refers to nucleic acids comprising the specifically defined sequences, it is preferred that the nucleic acid as a whole is a part of a gene or corresponds to an entire gene, but generally does not extend beyond the length of an entire gene. This will become apparent in view of preferred embodiments described further below. In particular, a nucleic acid in accordance with the first aspect does not extend to entire chromosomes.

Preferred levels of sequence identity are at least 85%, at least 90%, at least 95%, at least 98% or at least 99%.

Two nucleotide sequences can be aligned electronically using suitable computer programs known in the art. Such programs comprise BLAST (Altschul et al. (1990), J. Mol. Biol. 215, 403-410), variants thereof such as WU-BLAST (Altschul & Gish (1996), Methods Enzymol. 266, 460-480), FASTA (Pearson & Lipman (1988), Proc. Natl. Acad. Sci. USA 85, 2444-2448) or implementations of the Smith-Waterman algorithm (SSEARCH, Smith & Waterman (1981), J. Mol. Biol. 147, 195-197). These programs, in addition to providing a pairwise sequence alignment, also report the sequence identity level (usually in percent identity) and the probability for the occurrence of the alignment by chance (P-value). Programs such as CLUSTALW (Higgins et al. (1994), Nucleic Acids Res. 22, 4673-4680) can be used to align more than two sequences. Preferably, default settings of the respective computer programs as published are used. A preferred alignment program is BLAST *(loc. cit.)*.

To the extent the first aspect relates to nucleic acids the sequence of which is not identical to any of the entries of the sequence listing, at least 50% of the CpG dinucleotide motifs in accordance with the invention are present in said sequence. Preferably, at least 70%, at least 80%, at least 90% or all of the CpG dinucleotide motifs which are present in SEQ ID NO: 1 are also present in a sequence in accordance with item (b) and exhibiting 80% or more sequence identity to any one of the sequences of SEQ ID NO: 1.

Each of SEQ ID NOs: 1 to 46 as referred to herein is a subsequence of a given gene which subsequence comprises a CpG cluster. The genes, designated by art-established gene symbols, and their correspondence to the sequences of SEQ ID NOs: 1 to 46 are given in Table 1 below. Table 1 furthermore includes the performance measures fold change (F.C.), area under the curve (AUC), sensitivity and specificity of prediction. P-values are determined using a t-test corrected for multiple testing according to Benjamini Hochberg; F.C. indicates the log difference between case and control. AUC is area under curve derived from Receiver operator characteristics curve analysis; Sens indicates Sensitivity derived from ROC-curve analysis and Spec indicates Specificity.

**Table 1**

| Gene symbol | SEQ ID NO: | P.value | F.C. | AUC | Sens | Spec |
|---|---|---|---|---|---|---|
| PAX9 | 4 | 7.05E-13 | 14.58 | 0.88 | 0.95 | 0.80 |
| HOXD10 | 2 | 1.30E-10 | 12.68 | 0.87 | 0.94 | 0.79 |
| TBX20 | 3 | 2.33E-03 | 1.5 | 0.86 | 0.93 | 0.78 |
| CACNA1B | 1 | 1.97E-06 | 15.98 | 0.81 | 0.90 | 0.71 |
| ZIC1 | 5 | 2.05E-07 | 10.42 | 0.77 | 0.87 | 0.68 |
| SIM1 | 6 | 4.99E-08 | 9.65 | 0.76 | 0.86 | 0.67 |
| DLX1 | 7 | 7.04E-06 | 12.52 | 0.73 | 0.83 | 0.64 |
| LMX1A | 8 | 3.54E-07 | 12.36 | 0.75 | 0.85 | 0.66 |
| DLX6AS1 | 9 | 1.55E-07 | 11.57 | 0.83 | 0.92 | 0.74 |
| MIR196A1 | 10 | 3.54E-07 | 16 | 0.81 | 0.90 | 0.72 |
| FEZF2 | 11 | 1.90E-08 | 15.68 | 0.80 | 0.89 | 0.71 |
| STAG3 | 12 | 7.68E-05 | 1.77 | 0.75 | 0.85 | 0.65 |
| PTPRN2 | 13 | 6.03E-03 | 1.16 | 0.83 | 0.92 | 0.74 |
| BARHL1 | 14 | 8.38E-06 | 12.86 | 0.75 | 0.85 | 0.65 |
| CCDC140 | 15 | 8.33E-09 | 6.4 | 0.84 | 0.93 | 0.76 |
| CpG138 | 16 | 1.24E-07 | 13.52 | 0.80 | 0.89 | 0.70 |
| GATA2 | 17 | 1.55E-07 | 11.27 | 0.77 | 0.86 | 0.67 |
| ADCYAP1 | 18 | 9.88E-07 | 2.09 | 0.80 | 0.89 | 0.71 |
| TBX5 | 19 | 2.41E-06 | 9.17 | 0.72 | 0.82 | 0.63 |
| CpG38 | 20 | 1.30E-05 | 14.8 | 0.74 | 0.84 | 0.63 |

(continued)

| Gene symbol | SEQ ID NO: | P.value | F.C. | AUC | Sens | Spec |
|---|---|---|---|---|---|---|
| NECAB1 | 21 | 1.57E-05 | 8.82 | 0.74 | 0.84 | 0.64 |
| ADRB1 | 22 | 1.99E-05 | 13.63 | 0.72 | 0.83 | 0.62 |
| OTX2 | 23 | 2.52E-05 | 1.44 | 0.80 | 0.89 | 0.70 |
| N KX2-6 | 24 | 5.06E-05 | 2.32 | 0.77 | 0.87 | 0.67 |
| SIM1 | 25 | 1.11E-04 | 7.28 | 0.73 | 0.83 | 0.63 |
| DRD4 | 26 | 1.11E-04 | 11.74 | 0.70 | 0.81 | 0.58 |
| TBX15 | 27 | 1.42E-04 | 11.84 | 0.69 | 0.80 | 0.59 |
| TRH | 28 | 1.48E-04 | 1.42 | 0.72 | 0.82 | 0.61 |
| FBP1 | 29 | 1.71E-04 | 14.52 | 0.75 | 0.85 | 0.64 |
| CpG615 | 30 | 1.71E-04 | 1.1 | 0.80 | 0.89 | 0.71 |
| BCAN | 31 | 7.87E-04 | 1.34 | 0.84 | 0.92 | 0.76 |
| TP73 | 32 | 8.39E-04 | 14.16 | 0.70 | 0.81 | 0.59 |
| MIIP | 33 | 1.09E-03 | 0.41 | 0.60 | 0.72 | 0.49 |
| HOXC4 | 34 | 1.33E-03 | 8.04 | 0.68 | 0.79 | 0.58 |
| HOXA7 | 35 | 1.44E-03 | 2.76 | 0.81 | 0.90 | 0.71 |
| BST1 | 36 | 1.54E-03 | 2.11 | 0.74 | 0.85 | 0.64 |
| C1orf114 | 37 | 1.75E-03 | 14.65 | 0.67 | 0.78 | 0.55 |
| FOXG1 | 38 | 2.96E-03 | 13.52 | 0.64 | 0.75 | 0.52 |
| ATP4A | 39 | 5.40E-03 | 0.71 | 0.71 | 0.82 | 0.60 |
| NOTCH1 | 40 | 1.10E-02 | 3.84 | 0.69 | 0.81 | 0.58 |
| CpG80 | 41 | 1.21E-02 | 1.15 | 0.73 | 0.84 | 0.63 |
| N/A | 42 | 2.10E-02 | 1.61 | 0.77 | 0.88 | 0.67 |
| HOXA7 | 43 | 2.26E-02 | 2.25 | 0.78 | 0.88 | 0.67 |
| ZNF529 | 44 | 3.31E-02 | 0.05 | 0.55 | 0.67 | 0.43 |
| PRLHR | 45 | 4.96E-02 | 0 | 0.59 | 0.70 | 0.48 |
| ARL5C | 46 | 1.06E-01 | 0.15 | 0.58 | 0.67 | 0.49 |

To the extent reference is made to CpG numbers in this table, these are the numbers according to the UCSC browser (J Mol Biol.;196(2):261-82 (1987)).

Table 2 provides structural and functional information about certain particularly preferred genes.

**Table 2**

| Gene symbol | Description |
|---|---|
| PAX9 | This gene is a member of the paired box (PAX) family of transcription factors. Members of this gene family typically contain a paired box domain, an octapeptide, and a paired-type homeodomain. These genes play critical roles during fetal development and cancer growth. The specific function of the paired box 9 gene is unknown but it may involve development of stratified squamous epithelia as well as various organs and skeletal elements. |

(continued)

| Gene symbol | Description |
|---|---|
| HOXD10 | This gene is a member of the Abd-B homeobox family and encodes a protein with a homeobox DNA-binding domain. It is included in a cluster of homeobox D genes located on chromosome 2. The encoded nuclear protein functions as a sequence-specific transcription factor that is expressed in the developing limb buds and is involved in differentiation and limb development. Mutations in this gene have been associated with Wilm's tumor and congenital vertical talus (also known as "rocker-bottom foot" deformity or congenital convex pes valgus) and/or a foot deformity resembling that seen in Charcot-Marie-Tooth disease. |
| TBX20 | This gene encodes a T-box family member. The T-box family members share a common DNA binding domain, termed the T-box, and they are transcription factors involved in the regulation of developmental processes.<br>This gene is essential for haart development. Mutations in this gene are associated with diverse cardiac pathologies, including defects in septation, valvulogenesis and cardiomyopathy. Alternatively spliced transcript variants encoding different isoforms have been found for this gene. |
| CACNA1B | The protein encoded by this gene is the pore-forming subunit of an N-type voltage-dependent calcium channel, which controls neurotransmitter release from neurons. The encoded protein forms a complex with alpha-2, beta, and delta subunits to form the high-voltage activated channel. This channel is sensitive to omega-conotoxin-GVIA and omega-agatoxin-IIIA but insensitive to dihydropyridines. Two transcript variants encoding different isoforms have been found for this gene. |
| ZIC1 | This gene encodes a member of the ZIC family of C2H2-type zinc finger proteins. Members of this family are important during development. Aberrant expression of this gene is seen in medulloblastoma, a childhood brain tumor. This gene is closely linked to the gene encoding zinc finger protein of the cerebellum 4, a related family member on chromosome 3. This gene encodes a transcription factor that can bind and transactivate the apolipoprotein E gene. |
| SIM1 | SIM1 and SIM2 genes are Drosophila single-minded (sim) gene homologs. SIM1 transcript was detected only in fetal kidney out of various adult and fetal tissues tested. Since the sim gene plays an important role in Drosophila development and has peak levels of expression during the period of neurogenesis,it was proposed that the human SIM gene is a candidate for involvement in certain dysmorphic features (particularly the facial and skull characteristics), abnormalities of brain development, and/or mental retardation of Down syndrome. |
| DLX1 | This gene encodes a member of a homeobox transcription factor gene family similar to the Drosophila distal-less gene. The encoded protein is localized to the nucleus where it may function as a transcriptional regulator of signals from multiple TGF-(17) superfamily members. The encoded protein may play a role in the control of craniofacial patterning and the differentiation and survival of inhibitory neurons in the forebrain. This gene is located in a tail-to-tail configuration with another member of the family on the long arm of chromosome 2. Alternatively spliced transcript variants encoding different isoforms have been described. |
| LMX1A | This gene encodes a homeodomain and LIM-domain containing protein.<br>The encoded protein is a transcription factor that acts as a positive regulator of insulin gene transcription. This gene also plays a role in the development of dopamine producing neurons during embryogenesis. Mutations in this gene are associated with an increased risk of developing Parkinson's disease. Alternate splicing results in multiple transcript variants. |
| DLX6AS1 | DLX6-AS1 DLX6 antisense RNA 1. |

(continued)

| Gene symbol | Description |
|---|---|
| MIR196A1 | microRNAs (miRNAs) are short (20-24 nt) non-coding RNAs that are involved in post-transcriptional regulation of gene expression in multicellular organisms by affecting both the stability and translation of mRNAs. miRNAs are transcribed by RNA polymerase II as part of capped and polyadenylated primary transcripts (pri-miRNAs) that can be either protein-coding or non-coding. The primary transcript is cleaved by the Drosha ribonuclease III enzyme to produce an approximately 70-nt stem-loop precursor miRNA (pre-miRNA), which is further cleaved by the cytoplasmic Dicer ribonuclease to generate the mature miRNA and antisense miRNA star (miRNA*) products. The mature miRNA is incorporated into a RNA-induced silencing complex (RISC), which recognizes target mRNAs through imperfect base pairing with the miRNA and most commonly results in translational inhibition or destabilization of the target mRNA. The RefSeq represents the predicted microRNA stem-loop. |
| STAG3 | The protein encoded by this gene is expressed in the nucleus and is a subunit of the cohesion complex which regulates the cohesion of sister chromatids during cell division. A mutation in this gene is associated with premature ovarian failure. Alternate splicing results in multiple transcript variants encoding distinct isoforms. This gene has multiple pseudogenes. |
| PTPRN2 | The protein encoded by this gene is a member of the protein tyrosine phosphatase (PTP) family. PTPs are known to be signaling molecules that regulate a variety of cellular processes including cell growth, differentiation, mitotic cycle, and oncogenic transformation. This PTP possesses an extracellular region, a single transmembrane region, and a single intracellular catalytic domain, and thus represents a receptor-type PTP. The catalytic domain of this PTP is most closely related to PTPRN/IA-2beta. PTP and PTPRN are both found to be major autoantigens associated with insulin-dependent diabetes mellitus. Three alternatively splice transcript variants of this gene, which encode distinct proteins, have been reported. |

The subject-matter of the first aspect relates to novel cancer-associated CpG clusters. The diseased state is characterized by methylation for all genes in accordance with the present invention. By determining the methylation status of one or more of said cancer-associated CpG clusters or specific cytidine(s) within one or more CpG clusters, lung cancer or a predisposition therefor may be diagnosed. This is the subject of the second aspect of the present invention and is detailed further below. Nucleic acids in accordance with the present invention may be used as such, uses including the mentioned diagnostic applications. Alternatively, they may serve as starting point for amplification reactions, said amplification reactions being further detailed below, as well as for amplification independent methods of determining the methylation status, such amplification independent methods also being further detailed below. Certain methods of the present invention provide for amplification being specifically applied to methylated templates.

In a preferred embodiment, at least one, preferably all cytidines at positions
(4) 4, 38, 40, 45 and 83 in SEQ ID NO: 1 is/are methylated.

This preferred embodiment explicitly lists the cytidines comprised in CpG motifs, said CpG motifs comprised in a CpG cluster in accordance with the present invention. In case of SEQ ID NO: 1, it is particularly preferred that all cytidines specifically indicated, namely those at positions 4, 38, 40, 45 and 83 are methylated. In a less preferred embodiment, only one, only two, only three, only four, only five, only six, only seven, or only eight cytidines are methylated. It is understood that the latter statement, when referring to a given integer number, is only applicable to those CpG clusters which contain a number of CpG motifs which number of CpG motifs is larger than the specific integer number under consideration in the latter statement. To provide an example, in case of SEQ ID NO: 1, a specific embodiment relates to four out of the five CpG dinucleotides being methylated.

In general, and as noted above, methylation of the various CpG dinucleotides comprised in a given CpG cluster of the invention is correlated. Accordingly, the cytidines in all indicated CpG dinucleotide motifs will generally be fully methylated or methylated to very high degree in a sample obtained from a patient having lung cancer or from an individual having a predisposition to develop lung cancer. Said sample may be a lung cell from the tumor.

Alternatively, it may be taken from a metastasis of a patient suffering from lung cancer. Also, it may be a circulating tumor cell from a patient suffering from lung cancer.

Furthermore, the above applies *mutatis mutandis* to any of the measures of methylation status disclosed herein such as fold change. To explain further, any given measure of methylation determined for the CpG dinucleotide motifs within a given CpG cluster in accordance with the present invention in general will be correlated. Preferably, any given measure of methylation determined for CpG dinucleotides within a given CpG cluster do not vary by more than 20%.

In a second aspect, the present invention provides a method of diagnosing lung cancer or a predisposition therefor, said method comprising determining the methylation status of cytidine in

(4) at least one CpG dinucleotide in a nucleic acid comprising or consisting of the sequence from position 4 to position 84 of SEQ ID NO: 1;

wherein methylation of the cytidine(s) or increased methylation of the cytidine(s) as compared to a non-cancer subject is indicative of lung cancer or a predisposition therefor, and wherein any diagnostic method practiced on the human or animal body is excluded.

In a related aspect (third aspect), the present invention provides a method of monitoring treatment of lung cancer or of a predisposition therefor, said method comprising determining the methylation status of cytidine, cytidine being defined as in accordance with the second aspect, wherein no methylation of cytidine(s) or decreased methylation of the cytidine(s) as compared to a patient suffering from lung cancer or an individual having a predisposition therefor, respectively, is indicative of an amelioration of lung cancer or a decreased predisposition therefor, respectively, and wherein any diagnostic method practiced on the human or animal body is excluded.

Accordingly, while hypermethylation is indicative of the diseased state, a decrease of methylation or absence of methylation can be used as indicators of successful therapeutic treatment.

Determining a state as being methylated will in generally be sufficient for diagnostic purposes. In other words, a reference state for comparison is generally not compulsory. Yet, the second aspect of the present invention also presents the alternative of increased methylation as compared to a non-cancer subject. The non-cancer subject may be a single individual. Alternatively, the term "non-cancer subject" is a population average. The non-cancer state is characterized by low, preferably less than 40%, less than 30%, less than 20%, less than 10%, less than 5%, less than 2%, or less than 1% methylation. This applies to all genes or CpG clusters of the present invention, respectively. The term "non-cancer subject" embraces, but is not confined to healthy individuals. We note, though, that healthy individuals are preferred. Generally speaking, non-cancer subjects may also include diseased persons, provided that they do not suffer from lung cancer.

Since, as noted above, diagnosis may be performed with a sample drawn from a subject or patient, it is understood that diagnostic methods practiced on the human or animal body are excluded. Furthermore, we note that the actual process of drawing the sample does not fall under the terms of the present claims. As such, the methods of the present invention also do not extend to invasive or surgical methods.

The individual or subject may be a human or an animal, preferably a mammal. The term "individual" includes patients such as patients suffering from lung diseases, preferably lung cancer, as well as healthy or apparently healthy individuals. The terms "individual" and "subject" are used equivalently herein.

The term "lung cancer" has its art-established meaning and relates to any malignant disease affecting a tissue of the lung. Further information in this respect may be taken from the background section herein above and references cited therein. It is understood that in patients suffering from lung cancer, diseased cells include diseased cells in the lung, but are not confined thereto. Rather, diseased cells may also embrace cells from metastasis as well as circulating tumor cells. Any of these cells may be comprised in the sample to be analyzed using the methods of the present invention.

As detailed further below, the term "lung cancer" includes *inter alia* early stages. Not only early stages can be diagnosed, but furthermore a predisposition for developing lung cancer. In other words, also in the absence of clinical symptoms or where clinical symptoms are difficult to detect, the method of the present invention permits diagnosis.

The method is preferably effected with cell-free DNA. Cell-free DNA may be obtained from plasma. Cell-free DNA, as a matter of fact, is a constituent of plasma. Typical amounts as found in healthy and diseased persons are reported in the examples enclosed herewith.

At least in this preferred embodiment relating to a plasma sample, more invasive methods such as taking a biopsy are actually dispensable. Yet, the present invention may be worked using biopsies such as lung biopsies as well. Also, the method of the invention may be used for cell-free DNA testing, followed by confirmatory biopsy testing, wherein in at least one out of cell-free DNA testing and biopsy testing or in either case the respective samples (cell-free DNA; cells comprised in the biopsy) are analyzed with the methods in accordance with the present invention. Alternatively, the biopsy may be analyzed according to histological parameters.

To the extent the method of the second aspect provides for determining the methylation status of more than one CpG dinucleotide in a given CpG cluster, and/or a methylation status of more than one CpG cluster, it is understood that a diagnosis of lung cancer or a predisposition therefor is provided in those instances, where at least 70%, at least 80%, or at least 90%, preferably all CpG dinucleotides the methylation status of which is to be determined are in fact methylated. Determining the methylation status of more than one CpG dinucleotides is a means of increasing accuracy of the method of diagnosing in accordance with the second aspect. The above defined fold change is a preferred measure of the methylation status.

In a preferred embodiment of the second and third aspect, the cytidines the methylation status of which is to be determined, are those defined in accordance with the preferred embodiments of the first aspect herein above.

In a preferred embodiment of both first and second aspect, said nucleic acid comprises or consists of any one of SEQ ID NOs: 1, 10, 11, 47, 56, 57, 93, 101 and 102.

This preferred embodiment provides sequences of increasing length and comprising the respective CpG cluster located in the subsequences of SEQ ID NO: 1 as defined in accordance with the first aspect.

In a related aspect (fourth aspect), the present invention provides a nucleic acid comprising or consisting of (a) the sequence of SEQ ID NO: 137 or 183.

With regard to this embodiment, the following applies. As noted above, Cs are amenable to conversion to T, to the extent they are not methylated. The sequence of SEQ ID NO: 137 is a derivative of sequence 1 wherein all Cs are converted into Ts with the exception of those Cs which occur within CpG dinucleotide motifs specified in accordance with the present invention; see column "C/T converted (1)" in Table 3. In the sequences of SEQ ID NO: 183, all Cs have been converted into Ts in their entirety; see column "C/T converted (2)" in Table 3. The latter correspond to the complete absence of methylation in which case all Cs are amenable to conversion, for example by means of bisulfite. In terms of length, the sequences of SEQ ID NO: 137 as well as 183 correspond to SEQ ID NOs: 47, 56 and 57.

As noted above, also sequences where the results of conversion of a C is not a T, but instead a U are subject of the present invention.

We furthermore note that SEQ ID NOs: 1 to 46 define shorter subsequence within SEQ ID NOs: 47 to 92. While C/T as well as C/U converted sequences as disclosed above correspond in length to SEQ ID NOs: 47 to 92, it is understood that also subsequences of the C/T and C/U converted sequences which subsequences correspond to SEQ ID NOs: 1 to 46 are subject of the present invention. "Correspondence" in this case means identical length and sequence identity with exception of the converted positions. Furthermore, the first aspect of the present invention defines specific subsequences within SEQ ID NOs: 1 to 46. C/T and C/U converted sequences corresponding to these specific subsequences of SEQ ID NOs: 1 to 46 are also embraced by the present invention.

Table 3 below explains the correspondence between these sequences.

**Table 3**

| Gene symbol | PCR product | Medium length construct | Long construct | C/T converted (1) | C/T converted (2) |
|---|---|---|---|---|---|
| PAX9 | 4 | 47 | 93 | 137 | 183 |
| HOXD10 | 2 | 48 | 94 | 138 | 184 |
| TBX20 | 3 | 49 | 95 | 139 | 185 |
| CACNA1B | 1 | 50 | 96 | 140 | 186 |
| ZIC1 | 5 | 51 | 97 | 141 | 187 |
| SIM1 | 6 | 52 | - | 142 | 188 |
| DLX1 | 7 | 53 | 98 | 143 | 189 |
| LMX1A | 8 | 54 | - | 144 | 190 |
| DLX6AS1 | 9 | 55 | 99 | 145 | 191 |
| MIR196A1 | 10 | 56 | 100 | 146 | 192 |
| FEZF2 | 11 | 57 | 101 | 147 | 193 |
| STAG3 | 12 | 58 | 102 | 148 | 194 |
| PTPRN2 | 13 | 59 | 103 | 149 | 195 |
| BARHL1 | 14 | 60 | 104 | 150 | 196 |
| CCDC140 | 15 | 61 | 105 | 151 | 197 |
| N/A | 16 | 62 | 106 | 152 | 198 |
| GATA2 | 17 | 63 | 107 | 153 | 199 |
| ADCYAP1 | 18 | 64 | 108 | 154 | 200 |
| TBX5 | 19 | 65 | 109 | 155 | 201 |
| N/A | 20 | 66 | 110 | 156 | 202 |
| NECAB1 | 21 | 67 | 111 | 157 | 203 |
| ADRB1 | 22 | 68 | 112 | 158 | 204 |
| OTX2 | 23 | 69 | 113 | 159 | 205 |
| N KX2-6 | 24 | 70 | 114 | 160 | 206 |

(continued)

| Gene symbol | PCR product | Medium length construct | Long construct | C/T converted (1) | C/T converted (2) |
|---|---|---|---|---|---|
| SIM1 | 25 | 71 | 115 | 161 | 207 |
| DRD4 | 26 | 72 | 116 | 162 | 208 |
| TBX15 | 27 | 73 | 117 | 163 | 209 |
| TRH | 28 | 74 | 118 | 164 | 210 |
| FBP1 | 29 | 75 | 119 | 165 | 211 |
| N/A | 30 | 76 | 120 | 166 | 212 |
| BCAN | 31 | 77 | 121 | 167 | 213 |
| TP73 | 32 | 78 | 122 | 168 | 214 |
| MIIP | 33 | 79 | 123 | 169 | 215 |
| HOXC4 | 34 | 80 | 124 | 170 | 216 |
| HOXA7 | 35 | 81 | 125 | 171 | 217 |
| BST1 | 36 | 82 | 126 | 172 | 218 |
| C1orf114 | 37 | 83 | 127 | 173 | 219 |
| FOXG1 | 38 | 84 | 128 | 174 | 220 |
| ATP4A | 39 | 85 | 129 | 175 | 221 |
| NOTCH1 | 40 | 86 | 130 | 176 | 222 |
| N/A | 41 | 87 | 131 | 177 | 223 |
| N/A | 42 | 88 | 132 | 178 | 224 |
| HOXA7 | 43 | 89 | 133 | 179 | 225 |
| ZNF529 | 44 | 90 | 134 | 180 | 226 |
| PRLHR | 45 | 91 | 135 | 181 | 227 |
| ARL5C | 46 | 92 | 136 | 182 | 228 |

Each of the nucleic acid sequences in accordance with the present invention may be fused to a heterologous sequence.

In another preferred embodiment of the method in accordance with the second aspect, the methylation status of cytidines according to

(1) items (4) and (9);
(2) items (4) and (2);
(3) items (4) and (6);
(5) items (1) and (4);
(6) items (4) and (5);
(7) items (4), (2) and (9);
(8) items (4), (2) and (6);
(9) items (4), (9) and (10);
(11) items (4), (9) and (11);
(12) items (4), (5) and (9);
(13) items (4), (2) and (10);
(14) items (1), (4) and (9);
(15) items (4), (2) and (12);
(16) items (1), (2) and (4); or
(17) items (4), (2) and (5)
is to be determined wherein

(1) at least one CpG dinucleotide in a nucleic acid comprising or consisting of the sequence from position 7 to position 101 of SEQ ID NO: 4;

(2) at least one CpG dinucleotide in a nucleic acid comprising or consisting of the sequence from position 11 to position 71 of SEQ ID NO: 2;

(4) is as defined in any one of claims 3 to 7;

(5) at least one CpG dinucleotide in a nucleic acid comprising or consisting of the sequence from position 28 to position 69 of SEQ ID NO: 5;

(6) at least one CpG dinucleotide in a nucleic acid comprising or consisting of the sequence from position 39 to position 85 of SEQ ID NO: 6;

(9) at least one CpG dinucleotide in a nucleic acid comprising or consisting of the sequence from position 5 to position 71 of SEQ ID NO: 9;

(10) is at least one CpG dinucleotide in a nucleic acid comprising or consisting of the sequence from position 2 to position 60 of SEQ ID NO: 10;

(11) at least one CpG dinucleotide in a nucleic acid comprising or consisting of the sequence from position 22 to position 63 of SEQ ID NO: 11; and

(12) at least one CpG dinucleotide in a nucleic acid comprising or consisting of the sequence from position 45 to position 67 of SEQ ID NO: 12.

These preferred combinations of markers are also given in the table below together with their prediction performance. The performance measure in this case is the difference of AUC values: AUC (combination) - AUC (random). The term "random" designates a combination of two or three markers, respectively, which has been chosen at random from the markers of the invention and tested on the same serum data set.

**Table 4**

| MARKER 1 | MARKER 2 | MARKER 3 | AUC_diff |
|---|---|---|---|
| PAX9 | DLX6AS1 | | 0,1446281 |
| PAX9 | HOXD10 | | 0,13553719 |
| PAX9 | SIM1 | | 0,12093664 |
| HOXD10 | DLX6AS1 | | 0,11818182 |
| PAX9 | CACNA1B | | 0,1261708 |
| HOXD10 | ZIC1 | | 0,11763085 |
| PAX9 | PTPRN2 | | 0,10220386 |
| HOXD10 | MIR196A1 | | 0,11184573 |
| HOXD10 | SIM1 | | 0,11404959 |
| MIR196A1 | FEZF2 | | 0,11652893 |
| HOXD10 | CACNA1B | | 0,1077135 |
| DLX6AS1 | FEZF2 | | 0,10137741 |
| DLX6AS1 | MIR196A1 | | 0,10082645 |
| PAX9 | ZIC1 | | 0,09614325 |
| ZIC1 | SIM1 | | 0,07217631 |
| MIR196A1 | BARHL1 | | 0,07713499 |
| CACNA1B. | FEZF2 | | 0,07988981 |
| DLX6AS1 | PTPRN2 | | 0,08126722 |
| DLX1 | FEZF2 | | 0,07272727 |
| PAX9 | HOXD10 | DLX6AS1 | 0,1677686 |
| PAX9 | HOXD10 | SIM1 | 0,15619835 |
| PAX9 | DLX6AS1 | MIR196A1 | 0,15812672 |
| DLX6AS1 | MIR196A1 | FEZF2 | 0,16804408 |

(continued)

| MARKER 1 | MARKER 2 | MARKER 3 | AUC_diff |
|---|---|---|---|
| PAX9 | DLX6AS1 | FEZF2 | 0,16143251 |
| HOXD10 | SIM1. | MIR196A1 | 0,15922865 |
| PAX9 | ZIC1 | DLX6AS1 | 0,15179063 |
| HOXD10 | DLX6AS1 | MIR196A1 | 0,16101928 |
| PAX9 | HOXD10 | MIR196A1 | 0,14517906 |
| PAX9 | CACNA1B. | DLX6AS1 | 0,15881543 |
| PAX9 | HOXD10 | STAG3 | 0,15757576 |
| PAX9 | HOXD10 | CACNA1B | 0,14545455 |
| HOXD10 | MIR196A1 | BARHL1 | 0,15495868 |
| HOXD10 | MIR196A1 | FEZF2 | 0,14958678 |
| HOXD10 | ZIC1 | MIR196A1 | 0,1476584 |
| PAX9 | HOXD10 | ZIC1 | 0,1399449 |
| HOXD10 | ZIC1 | DLX6AS1 | 0,13663912 |
| HOXD10 | ZIC1 | SIM1 | 0,13719008 |
| HOXD10 | SIM1. | FEZF2 | 0,13471074 |
| PAX9 | DLX6AS1 | STAG3 | 0,13829201 |
| PAX9 | HOXD10 | DLX1 | 0,13939394 |

As noted above, the determination of the methylation status of more than one cytidine, is in general a means of increasing accuracy of diagnosis. The subject-matter of the above preferred embodiments are specific combinations of CpG clusters which were found to provide particularly accurate diagnosis.

In a further preferred embodiment of the method of the invention (a) the methylation status of no further nucleotides is to be determined; or (b) said method furthermore comprises determining the methylation status of cytidine in at least on CpG dinucleotide in a nucleic acid comprising or consisting of the sequence of SEQ ID NO: 321, wherein methylation of cytidines or increased methylation of cytidines as compared to a non-cancer subject is indicative of lung cancer, cytidines being those defined herein above and cytidine(s) in the sequence of SEQ ID NO: 321.

More generally speaking, while the use of the sequence of SEQ ID NO: 321 is preferred, it is understood that not only nucleic acids comprising or consisting of the sequence of SEQ ID NO: 321 may be used, but instead any nucleic acid comprising or consisting of the sequence of Shox2 or a subsequence thereof, said subsequence having a minimal length of 50 bases.

This embodiment provides two alternatives. In the first alternative (a) it is established that the cytidines identified in the preceding embodiments constitute a closed list. In the alternative (b) the determination of cytidine methylation status may be extended to one or more further genes, a particularly preferred further gene being Shox2. To the extent the methylation status of one or more cytidines in the Shox2 sequence is to be determined, it is understood that diagnosis is based on the methylation status of all cytidines analyzed, namely those in accordance with the preceding embodiments together with those in the Shox2 sequence.

In a further preferred embodiment, said determining is effected in a sample taken from a subject, said sample preferably being a bodily fluid, more preferably plasma, serum or sputum; a biopsy taken from said cancer; a biopsy taken from a metastasis; or a circulating tumor cell.

In a particularly preferred embodiment, prior to said determining, (a) cell-free DNA comprised in said sample is enriched or isolated; (b) cell-free DNA is subjected to digestion with one or more methylation-sensitive restriction enzymes; and/or (c) cell-free DNA is pre-amplified.

Methods for enriching or isolating DNA from a sample are well-known in the art. A preferred method is described in the examples enclosed herewith.

Digestion in accordance with item (b) is preferably effected by bringing enriched or isolated cell-free DNA into contact with one or more of the mentioned enzymes. Alternatively, a sample, as it is obtained from a subject, may be brought into contact with said enzymes. Preferred enzymes are disclosed further below. Digestion with methylation-sensitive restriction enzymes is a particularly advantageous approach for analyzing the methylation status of DNA. In a specific example, the high multiplexing properties of MSRE digested DNA allow to perform 96 qPCR assays in parallel on cfDNA derived from only 400µl patient serum. Methylation sensitive restriction enzymes are characterized in that they are not able to cleave at the normal cleavage site in those cases where the cytidines comprised in the cleavage site are methylated.

Item (c) provides for a pre-amplification step. Pre-amplification is typically applied after enzymatic digestion. It is a means of enriching and/or increasing the amount of methylated DNA, in particular prior to the subsequent actual step of determining, preferably by amplification, which yields the desired readout.

In a further preferred embodiment, said determining is performed by one or more of the following procedures: (a) a support-based methylation assay; (b) amplification, preferably PCR, more preferably conventional or real-time PCR, of DNA digested in accordance with the invention and optionally pre-amplified in accordance with the invention; (c) a methylated CpG island recovery assay; (d) a bisulfite assay or bisulfite sequencing; (e) a proximity hybridization assay; and (f) nanopore sequencing. Bisulfite-based methods are reviewed in Egger et al., Expert Rev. Mol. Diagn. 12, 75-92 (2012).

This preferred embodiment includes the above-mentioned option of amplification for determining the methylation status, together with other alternatives. The other alternatives are known in the art. As an example, we refer to Rauch et al. (loc. cit.), where a methylated CpG island recovery assay is described. Further art-established methods for determining the methylation status include MSRE (or HELP), MSP, qMSP, MethyLight, HeavyMethyl, methyl-BEAMing, COBRA, MBD, MEDIP, MLPA, Maldi TOF and McBC (i.e.CHARM).

In a particularly preferred embodiment, (a) cell-free DNA comprised in said sample is isolated; (b) the isolated DNA obtained in (a) is subjected to digestion with one or more methylation-sensitive restriction enzymes; (c) the digested DNA obtained in (b) is pre-amplified; (d) the pre-amplified DNA obtained in (c) is amplified.

In an alternative preferred embodiment, said methylation status is determined without any amplification. This can be done, for example, by nanopore sequencing. This is a means of directly detecting methylated as well as hydroxymethylated cytidine which does not require a hybridization assay either. Other amplification independent methods of determining the methylation status rely on detecting methylated and/or hydroxymethylated cytidines with antibodies or aptamers specific for methylated and hydroxymethylated cytidines, respectively. Other proteins specifically binding such modified cytidines are methyl-binding domain proteins. Upon binding of any of said binders (antibodies, aptamers, methyl-binding domain proteins), proximity hybridization assays may be used in order to determine which positions are methylated.

Enriching or isolating of DNA prior to any of the above amplification independent methods is preferred, but not required. Similarly, the above disclosed amplification independent methods may be used in combination with digestion by methylation-sensitive restriction enzymes, but may also be used without prior digestion.

This embodiment defines a particularly preferred sequence of steps leading from a sample, preferably a plasma sample, to amplified DNA, wherein the amplified DNA, owing to the use of restriction enzymes in accordance with step (b) of this particularly preferred embodiment, consists of or is enriched with regard to methylated DNA.

In principle, any methylation sensitive restriction enzyme may be used. Preferably, said methylation-sensitive restriction enzymes are selected from Hpall, HinP1I, Acil and HpyCH4IV.

So far, the methylation status of cytidines has been described herein as a disease-relevant parameter useful for diagnosis of lung cancer. In addition thereto, the amount of cell-free DNA comprised in the sample, preferably a serum sample, may be used as a further predictive parameter.

Accordingly, in a preferred embodiment, furthermore the amount of cell-free DNA in said sample is determined.

In a particularly preferred embodiment, an elevated amount of cell-free DNA as compared to a healthy subject is indicative of disease.

To the extent both methylation status of cytidines and the amount of cell-free DNA are used in the method of diagnosis of the second aspect of the present invention, provided below are specific combinations of parameters. In a preferred embodiment (a) the methylation status of cytidine(s) according to (1) item (1) as defined in the second aspect of the invention; (2) item (2) as defined in the second aspect of the invention; (3) items (1) and (2) as defined in the second aspect of the invention; (4) items (1) and (9) as defined in the second aspect of the invention; (5) items (2) and (5) as defined in the second aspect of the invention; or (6) items (1) and (4) as defined in the second aspect of the invention; and (b) the amount of cell-free DNA in said sample are determined.

In a preferred embodiment of the method of the second aspect, said lung cancer is selected from adenocarcinoma, squamous cell carcinoma, small cell lung carcinoma, large cell lung carcinoma, early stage lung cancer (stage I or II) and late stage lung cancer (stage III or IV).

In a fifth aspect, the present invention provides a kit comprising or consisting of the following set of primers:

> (1) nucleic acids comprising or consisting of the sequences of SEQ ID NO: 229 and 230 or sequences having at least 80% sequence identity thereto.

The two sequences specified in each item of the third aspect are forward and reverse primer useful for amplifying the sequence of a given CpG cluster. The order of items corresponds to the order of items in the first aspect.

Preferred levels of sequence identity are given herein above as well as means and methods for determining sequence identity.

In a preferred embodiment of the kit of the invention, the kit furthermore comprises one or more of the following: (a) a manual with instructions for performing the method of the second aspect; (b) one or more buffered solutions or reagents for the preparation thereof; (c) one or more reaction vessels.

Furthermore comprised within the kit of the present invention, for example as part of the manual, may be information about threshold values as defined further above. Such threshold values are useful to distinguish between diseased and non-diseased states and render the use of control samples dispensable.

If not expressly indicated otherwise, preferred embodiments of one particular aspect of the present invention give rise to corresponding preferred embodiments of all other aspects.

The above considerations apply *mutatis mutandis* to all attached claims.

The figures show:

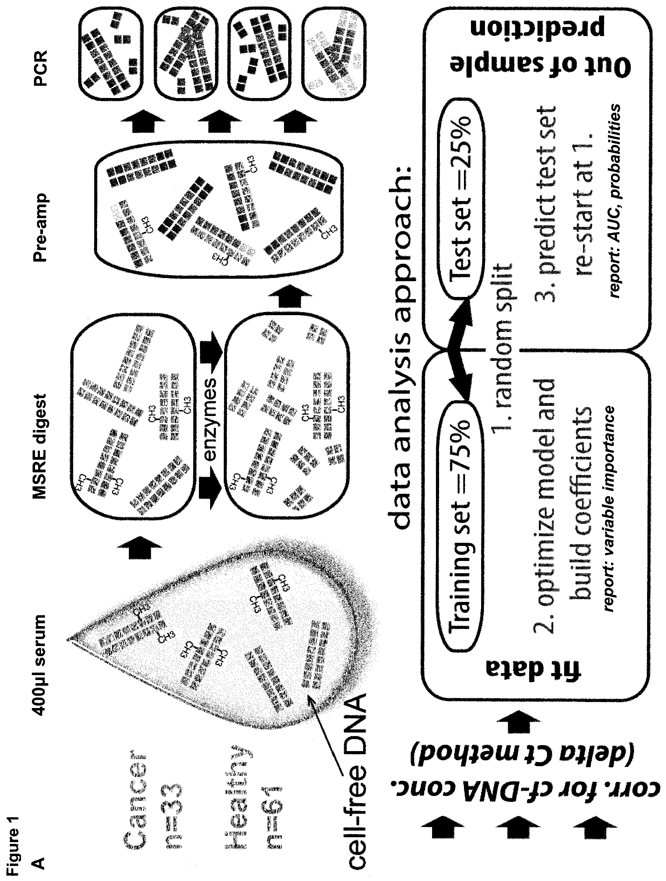

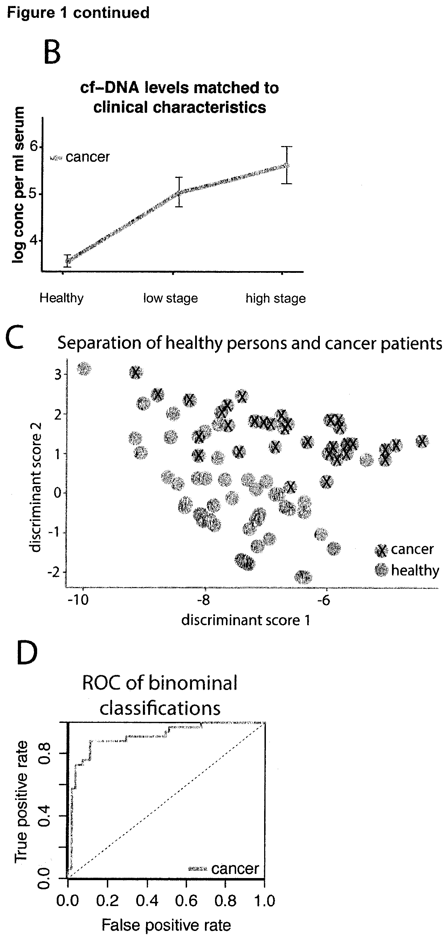

Figure 1: Overview of cfDNA analysis.

(A) cfDNA processing workflow. Colored strings represent cfDNA with attached $CH_3$ indicating methylated DNA. Color represents DNA origin: purple from healthy tissue red from neoplastic tissue. Methyl-sensitive restriction enzyme (MSRE) digest is the key step of the workflow: the unmethylated DNA gets digested and only methylated DNA serves as a target in the preamplification step (Pre-amp) of the PCR. Additional to already existing color-code newly introduced colors indicate PCR-primers for methylation target loci and grey color indicates amplified DNA. One multiplexed preamplification is performed per sample, which subsequently is split into single reactions for qPCR detection (PCR). The lower panel shows our prediction and resampling approach, which is described in more detail in the supplemental material. (B) Isolated cfDNA per ml serum/plasma. Values are log2 transformed. Error bars indicate the standard deviation of the mean. Low stage cancer represents patients diagnosed with cancer of stage I or II, high stage indicates stage III or IV. (C) Fisher discriminant analysis was performed using the top 30 markers. Subsequently the data were projected to the 2 most informative projection directions (discriminant scores). The plot shows the separation of patient samples based on the transformed data, which may be interpreted similarly to a Principal Component Analysis. (D) ROC-curve analysis shows the quality of separation of each analyzed disease versus healthy controls.

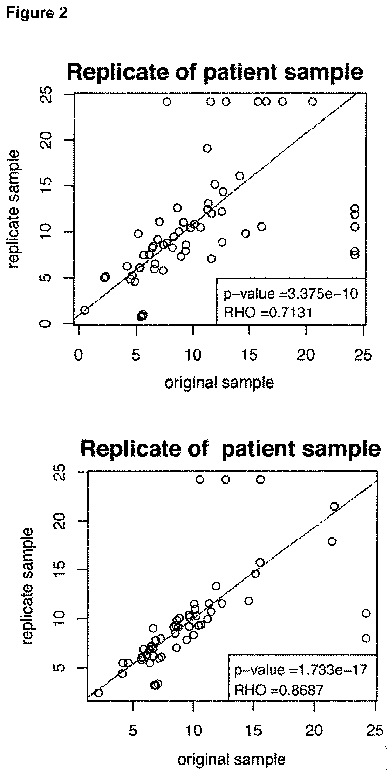

Figure 2: Delta Ct normalized values of two representative samples (out of 16). Replicates were introduced at the level of cf-DNA extraction from serum; subsequently samples were manipulated in two independent experimental fractions. P-value reflects Spearman P-value of correlation. Red line indicates linear regression line of data points. Double negative values (i.e. no PCR signal in both samples) were removed before analysis, to leave spearman's rho and P value unbiased.

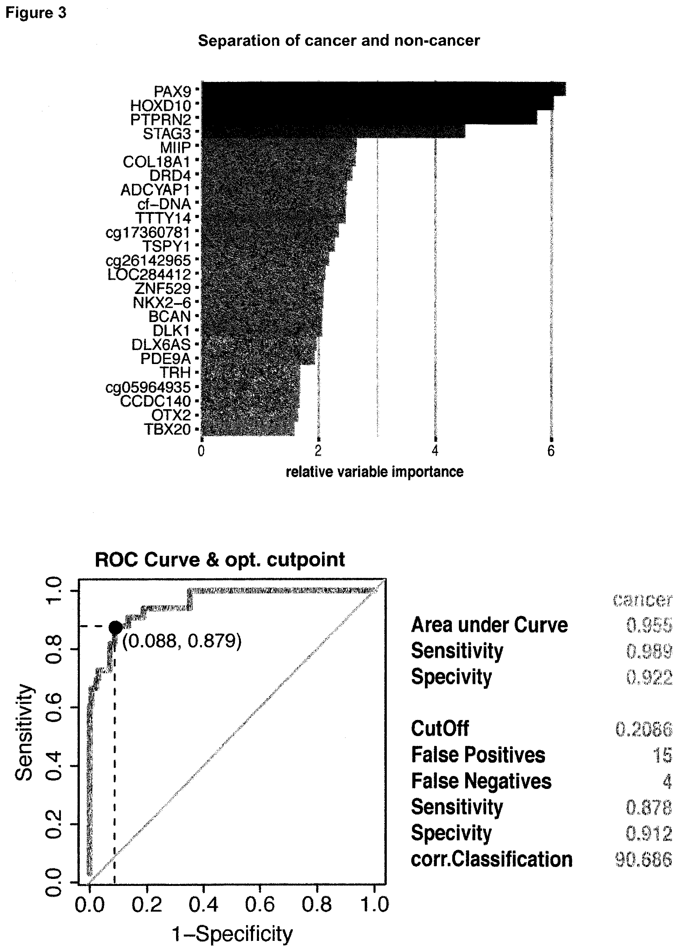

Figure 3: Differential diagnosis approach, where each disease is separated from all patient samples. Bar plots indicate the relative variable importance for each model. Model distinguishes between cancer and residual samples. The relative variable importance reflects the contribution of each variable to the prediction of the sample membership. ROC curve indicates the quality of separation including the chosen cut off value, which would be used for the diagnosis of future patient samples. Values at cut points reflect achieved specificity and sensitivity for the chosen cut off value. The rightmost panels summarizes the ROC curve analysis starting with the values derived from conventional ROC curve analysis. The section below, starting with "CutOff", lists the values gained by the application of the specific cut off values.

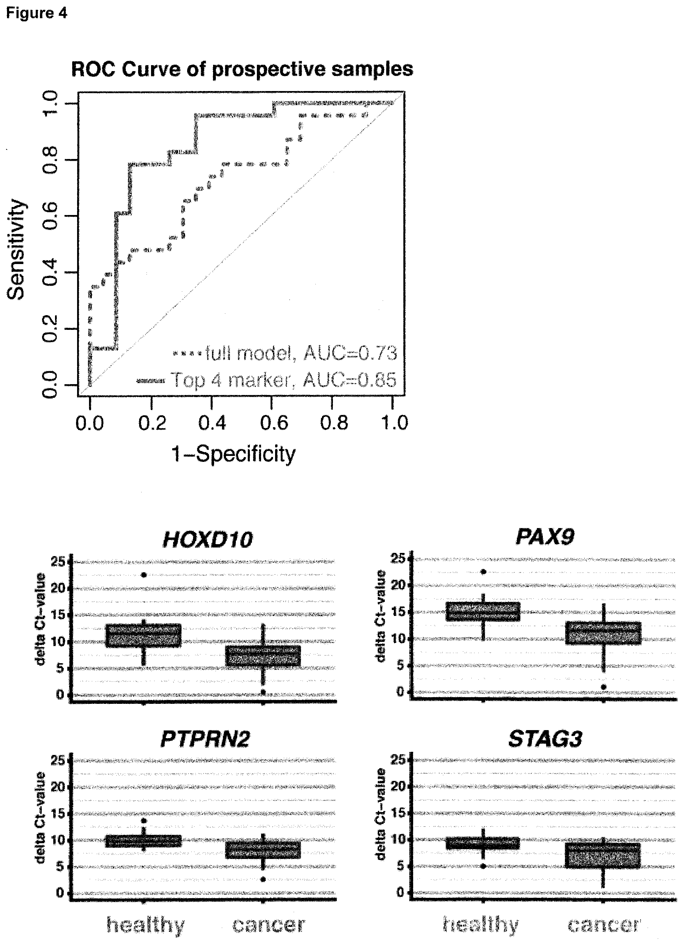

Figure 4: Proof of Principle: Prospective sample prediction. ROC curve analysis of 46 serum samples, which were classified in a prospective analysis setting. Prediction is based on coefficients derived from the model presented Figure 2. The dashed line represents the separation of cancer and non-cancer patients, using all 64 variables. The solid line represents the prediction based on the top 4 markers. Boxplots show the normalized Ct values of these top 4 markers.

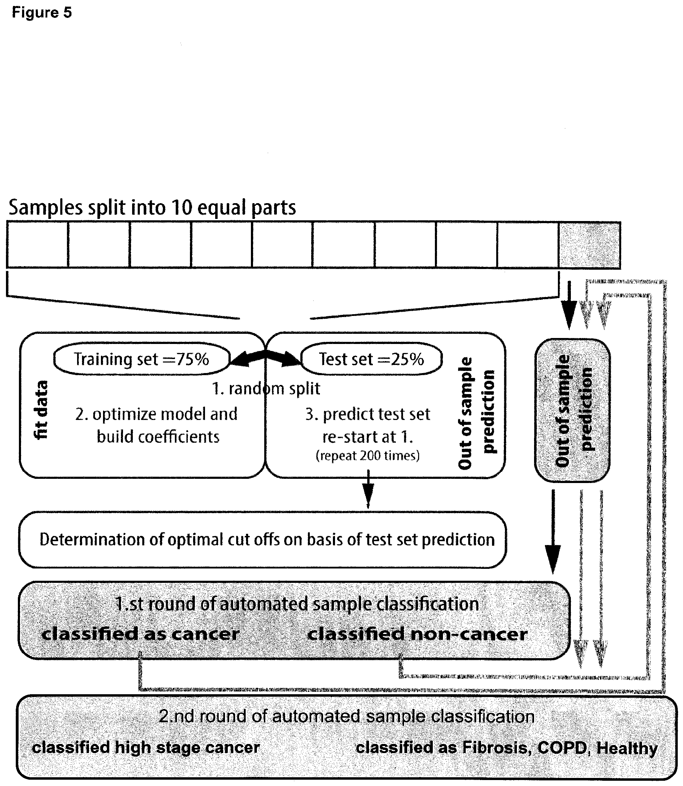

Figure 5: Scheme to simulate prospective sample data.

The examples illustrate the invention.

Example 1

Materials and Methods

*Patients*

**Table 5: Clinical Characteristics of patient serum/plasma samples.**

|  | Original set | PoP set |
|---|---|---|
|  | Patients | Patients |
| **Healthy** | **N=61 (29.9%)** | **N=23 (50%)** |
| healthy | n=27 (13.2%) | n=23 (50%) |
| COPD at risk | n=34 (16.6%) | - |
| **Lung Cancer** | **N=33 (16,1%)** | **N=23 (50%)** |
| low stage (I-II) | n=9 (4.4%) | n=8 (17.4%) |
| High stage (III-IV) | n=15 (7.3%) | n=12 (26%) |
| **Lung Cancer** | **N=33 (16,1%)** | **N=23 (50%)** |
| AdenoCa | n=11 (5.4%) | n=9 (19.5%) |
| SqCC | n=8 (3.9%) | n=4 (8.6%) |
| SCLC | n=7 (3.4%) | n=6 (13%) |
| LCLC | n=7 (3.4%) | n=4 (8.7%) |
| **COPD** | **N=42 (20.5%)** | - |
| GOLD 1-2 | n=31 (15.1%) | - |
| GOLD 3-4 | n=11 (5.4%) | - |
| **Fibrosis** | **N=68 (33.3%)** | **-** |
| UIP | n=27 (13.2%) | - |
| NSIP | n=11 (5.4%) | - |
| HP | n=22 (10.7%) | - |

In sum, 250 serum samples were analyzed. 204 within the original set and 46 within the PoP (proof of principle) set. Information on the characteristic histopathologic pattern of IPF was not available for 8 fibrosis patients and cancer staging information was not available for 7 sera of the original set and 3 sera of the PoP set. AdenoCa, lung adenocarcinoma; SqCC: squamous cell carcinoma, SCLC: small cell lung cancer, LCLC: large cell lung cancer.

*Cf-DNA Isolation*

Cell free DNA was isolated using Roche High pure template preparation kit (Roche) with a modified isolation protocol published previously (Wielscher, et al., BMC Clin Pathol 11, 11 (2011)). DNA was eluted in 55$\mu$l. After DNA concentration measurement with Pico Green (Invitrogen) of 1$\mu$l sample, the residual volume was reduced to approximately 10$\mu$l using Microcon-10 Centrifugal Filters Ultracel YM-10 (Millipore) according to manufacturer's instruction.

*MSRE digestion (methylation sensitive step*

To be able to distinguish between methylated and unmethylated DNA the isolated cell free DNA was digested with four methylsensitive restriction enzymes namely Hpall (ThermoFisher), Hin6I (ThermoFisher), Acil (NewEnglandBiolabs) and HpyCHIV4 (NewEnglandBiolabs). The reaction was carried out in a total volume of 12$\mu$l consisting of 8$\mu$l sample material, 1.2$\mu$l reaction buffer Tango (ThermoFisher), 0.4$\mu$l of each restriction enzyme (Hpall, Hin6I, Acil, HpyCHIV4 each 10U/$\mu$l) and 1.2$\mu$l water. Reaction was incubated at 37°C for 16h and followed by heat inactivation.

*Pre-amplification (multiplexed amplification of 96 targets per sample)*

10μl of MSRE digestion reaction was subjected to multiplexed pre-amplification step. Preamplification reaction was performed in a volume of 20μl [..] 25nM of each pair of the primer pairs were added per reaction. 19 cycles of preamplification were performed. Subsequent to amplification samples were diluted 1:5 with water to avoid primer contamination in the qPCR reaction.

*qPCR read out (in nl scale on Fluidigm Biomark)*

96.96 Dynamic Array™ IFC chips for Gene Expression (Fluidigm) were primed at an ICF controller HX (Fluidigm). The sample mix was 3 μl 2 x TaqMan GeneExpression MasterMix (ABI), 0.3 μl 20x DNA Binding Dye Sample Loading Reagent (Fluidigm), 0.3 μl 20x EvaGreen Binding Dye (Biotium), 0.9 μl of water and 1.5 μl of the preamplified sample. The assay mix was 3 μl 2x Assay Loading Reagent (Fluidigm), 0.3 μl water and 2.7 μl primermix (20 μM per primer pair). 6μl sample mix and 6μl assay mix were loaded to Fluidigm Priming station. The qPCR was performed by BioMark™ HD Reader (Fluidigm). The thermal mixing and UNG activation was 50°C for 120 sec, at 70 °C for 1800 sec and 25°C for 600 sec; followed by 120 sec at 50°C for 120. The PCR was 40 cycles of 95°C for 15 sec and 60 sec at 68°C followed by a temperature gradient from 65°C to 95°C for melting curve analysis.

*Data analysis*

Ct value preprocessing and data normalization

Prior to delta Ct normalization and supervised data analysis assays that produced less than 50 datapoints (out of 204 samples) were excluded. A total of 29 assays were excluded before subsequent analysis, which lead to 63 predictor variables for presented analysis. PCR fragments (i.e. Ct-values) exhibiting a Tm variance above +/- 1.5 were excluded from further data analysis. On the sample side no sample had to be excluded as all 204 showed at least three positive internal controls.

Data were corrected for different cf-DNA concentrations by delta Ct normalization. For delta Ct normalization the geometric mean of the Ct values produced by assays for the SNRPN, H19, JUB and IRF4 locus were subtracted from the Ct values produced by the 63 predictor variables (Supplemental table 7). Missing values due to the lack of PCR signal of qPCR assay for several samples were replaced by 40 (i.e. number of PCR cycles) minus the geometric mean of internal controls of all analyzed samples (i.e. 24.233). After delta Ct normalization the log2 transformed cell free DNA concentration values were added to the dataset.

Data analysis approach

To avoid over fitting of the data exclusively probabilities derived from an out of sample prediction are reported in the present study. This was achieved as follows. The 192 samples (outlying observation excluded from training set) were split randomly into 2 parts, a training set consisting of approximately 75% of all patients' samples and test set consisting of 25% of the 192 samples. The training set was used to build a fitted model of PCR data yielding coefficients for the 64 predictor variables (incl. log2 transformed cf-DNA concentration). Subsequently the test set was predicted on basis of the 64 coefficients computed from the training set (Figure 1A). This simply represents the scheme of an out of sample prediction, because none of the test set samples was used to fit the data. The procedure was repeated 200 times, to assure that every sample was is represented in the test set prediction. On average each sample was present in the test set 26 times. Thus gained approximately 26 probabilities of each sample for their diagnostic group membership were averaged before subsequent analysis. On basis of the test set prediction the correct classification rates and ROC-curve analysis was performed. On basis of training sets the variable importance was determined.

Lasso penalized logistic Regression

Binominal classification comparing each disease group to normal controls (Figure 1) was performed on basis of linear models using lasso penalized logistic regression taken from the R package glmnet. The optimal lambda value was determined by a 5 fold cross validation within training set. This determination process was optimized towards the maximum AUC accessible for the separation of case and control. If the number of cases was not equal to the number of controls weights to compensate the imbalance were assigned.

$$weights_{cases} = \frac{N_{all\ samples} \times \frac{1}{2}}{N_{cases}} \qquad \& \qquad weights_{contr.} = \frac{N_{all\ samples} \times \frac{1}{2}}{N_{contr.}}$$

Thus specified lambda values and resulting coefficients were used to perform the out of samples prediction.

Visualization of patient group separation

The Fisher discriminant scores in Figure 1C were produced as follows. To reduce noise for graphical display of data exclusively the top 30 variables distinguishing between diseased persons and controls were used for computation of projection directions. A fisher discriminant analysis was performed, where projection directions of the maximum separation between the sample groups based on the top 30 variables were identified and plotted. The plot shows the transformed data, which may be interpreted similar to a Principal Component analysis.

Generalized boosted regression models

Differential multinomial classifications were performed using boosted trees method. Here the idea of gradient boosting, where a number of weak classifiers are combined to a powerful committee, which is achieved by specific weighting of the samples, is applied to classification trees (Friedmann, Annals of Statistics, 1189-1232 (2001)). In the present study the gbm function build trees with at least 6 observations per terminal node of each tee. The optimal iteration was determined by 5 fold cross validation, in sum 4000 trees were computed (i.e. iterations) with a learning rate of 0.001. Best iteration of the learning process was used to predict the test set samples.

Determination of optimal cutpoints

The optimal cut off values for diagnostic purposes were determined on basis of predicted averaged group membership probability derived from test set predictions. This was achieved using the R package OptimalCutpoints. As criteria to appoint the optimal cut off value Youden's Index was used for separation of cancer samples.

Software

All computations and statistical analyses were performed using R 3.0.2 (http://www.r-project.org) (Team, R foundation of Statistical Computing (2005)) and Bioconductor 2.13 (http://www.bioconductor.org) (Gentleman, Genome biology, 5, R80 (2004)).

Example 2

<u>Results</u>

All patients 204 patients from the original set and 46 patients from the PoP set are given in Table 1. Herein presented are the results derived from normal controls (i.e. healthy persons and COPD Gold stage 0) and cancer patients. Markers and prediction models specific for fibrosis or COPD are not discussed in the following section.

*cfDNA amounts in patient sera*

No difference in total cfDNA concentrations between serum and plasma was measured. In healthy controls 12.5 ng/ml were isolated on average. The most pronounced cfDNA increase was observed in cancer patients (Figure 1B). In early stage (i.e. stage I and stage II) cancer patients the cfDNA amount increased 2.8 fold to 35.6ng /ml, in late stage cancer patients (stage III, stage IV) an average of 47.4 ng/ml was observed.

*cfDNA reveals disease specific methylation signature*

To overcome the challenging task of cfDNA methylation analysis in patient serum, a MSRE based approach was chosen (Figure 1A). To introduce a difference between methylated and unmethlyated cfDNA, serum isolates were digested with a mix of four different methyl sensitive restriction enzymes, which fragmented the unmethylated DNA and leave the methylated DNA fraction intact. Digested DNA was then preamplified in a reaction with 96 primer pairs. Finally qPCR read out was performed in 6nl single reactions (Figure 1A). In total, 204 serum/plasma samples were randomized before DNA isolation step and processed in 3 independent experimental fractions. A mean Spearman's rank correlation coefficient of 0.69(+/- 0.158) was observed analyzing 16 replicate samples distributed to all three experimental fractions, indicating the high reproducibility of the applied method.

On the basis of differential methylation observed in patient serum, a penalized logistic regression was performed. Therefore, the qPCR Ct values of 63 candidate loci and the cfDNA amounts were used to create a model based on the training set (75% of samples) and prediction was performed on the remaining 25% of the samples, this process was repeated 200 times and the resulting probabilities of sample class membership were averaged (Figure 1A). To estimate the diagnostic value of tested methylation marks, each disease group was compared to healthy (Figure 1D). An area under curve of 0.91 for cancer patients versus healthy controls was measured. Quality of separation of diseased patients from normal controls are also illustrated as the top 2 projection directions derived from the 30 most frequent modeled variables that underwent a Fisher discriminant analysis (Figure 1C).

*Cancer classification*

As depicted in Figure 3 regression tree based gradient boosting, embedded in the same resampling strategy as described in the previous section, outperformed comparable methods like L1 regression, support vector machines or random forests. This process yielded a stable model and an area under curve of 0.95, which was achieved when separating all non-cancer patients from cancer patients (Table 1).

To assess the quality and the stability of the methylation markers, an independent sample set was analyzed (PoP-set, table 1). The samples were analyzed based on the models given in figure 2 embedded into the herein presented automated prediction procedure, which results in a prospective analysis setting where the novel samples (PoP set) were used solely to get predicted and had no influence on the model building process. The ROC curve analysis (Figure 4) shows that the smaller model consisting of the top 4 markers (Figure 2B) outperforms the full model yielding an AUC of 0.85 with a sensitivity of 0.97 and specificity of 0.73 (Figure 4). Although only cancer and normal sera were available for the PoP-set, the whole analysis pipeline was tested and a separation of the PoP samples into all possible groups was performed. Solely one cancer case was not classified as cancer, 13 healthy controls were correct classified, whereas two healthy cases were rated as COPD patients and 8 healthy controls as cancer.

**Claims**

1. A nucleic acid comprising or consisting of

    (a) the sequence
    (4) from position 4 to position 84 of SEQ ID NO: 1, wherein the cytidine in at least one CpG dinucleotide comprised in said sequence is methylated;
    or
    (b) a sequence heaving at least 80% sequence identity to a sequence of (a) wherein the cytidine in at least one CpG dinucleotide comprised in said sequence is methylated.

2. The nucleic acid of claim 1, wherein at least one, preferably all cytidines at positions (4) 4, 38, 40, 45 and 83 in SEQ ID NO: 1 is/are methylated.

3. A method of diagnosing lung cancer or a predisposition therefor, said method comprising determining the methylation status of cytidine in
    (4) at least one CpG dinucleotide in a nucleic acid comprising or consisting of the sequence from position 4 to position 84 of SEQ ID NO: 1;
    wherein methylation of the cytidine(s) or increased methylation of the cytidine(s) as compared to a non-cancer subject is indicative of lung cancer or a predisposition therefor, and wherein any diagnostic method practiced on the human or animal body is excluded.

4. A method of monitoring treatment of lung cancer or of a predisposition therefor, said method comprising determining the methylation status of cytidine, cytidine being defined as in claim 3, wherein no methylation of cytidine(s) or decreased methylation of the cytidine(s) as compared to a patient suffering from lung cancer or an individual having a predisposition therefor, respectively, is indicative of an amelioration of lung cancer or a decreased predisposition therefor, respectively, and wherein any diagnostic method practiced on the human or animal body is excluded.

5. The method of claim 3 or 4, wherein the cytidine(s) the methylation status of which is to be determined are those defined in claim 2.

6. The nucleic acid of claim 1 or 2, or the method of claim 3 or 4, wherein said nucleic acid comprises or consists of any one of SEQ ID NOs: 1, 10, 11, 47, 56, 57, 93, 101 and 102.

7. A nucleic acid consisting of the sequence of SEQ ID NO: 137 or 183.

8. The method of any one of claims 3 to 5, wherein the methylation status of cytidines according to

(1) items (4) and (9);
(2) items (4) and (2);
(3) items (4) and (6);
(5) items (1) and (4);
(6) items (4) and (5);
(7) items (4), (2) and (9);
(8) items (4), (2) and (6);
(9) items (4), (9) and (10);
(11) items (4), (9) and (11);
(12) items (4), (5) and (9);
(13) items (4), (2) and (10);
(14) items (1), (4) and (9);
(15) items (4), (2) and (12);
(16) items (1), (2) and (4); or
(17) items (4), (2) and (5)

is to be determined, wherein said items are defined as follows:
cytidine in

(1) at least one CpG dinucleotide in a nucleic acid comprising or consisting of the sequence from position 7 to position 101 of SEQ ID NO: 4;
(2) at least one CpG dinucleotide in a nucleic acid comprising or consisting of the sequence from position 11 to position 71 of SEQ ID NO: 2;
(4) as defined in any one of claims 3 to 7;
(5) at least one CpG dinucleotide in a nucleic acid comprising or consisting of the sequence from position 28 to position 69 of SEQ ID NO: 5;
(6) at least one CpG dinucleotide in a nucleic acid comprising or consisting of the sequence from position 39 to position 85 of SEQ ID NO: 6;
(9) at least one CpG dinucleotide in a nucleic acid comprising or consisting of the sequence from position 5 to position 71 of SEQ ID NO: 9;
(10) at least one CpG dinucleotide in a nucleic acid comprising or consisting of the sequence from position 2 to position 60 of SEQ ID NO: 10;
(11) at least one CpG dinucleotide in a nucleic acid comprising or consisting of the sequence from position 22 to position 63 of SEQ ID NO: 11; and
(12) at least one CpG dinucleotide in a nucleic acid comprising or consisting of the sequence from position 45 to position 67 of SEQ ID NO: 12.

9. The method of any one of claims 3 to 5 and claim 8, wherein

(a) the methylation status of no further nucleotides is to be determined; or
(b) said method furthermore comprises determining the methylation status of cytidine in at least one CpG dinucleotide in a nucleic acid comprising or consisting of the sequence of SEQ ID NO: 321, wherein methylation of cytidines or increased methylation of cytidines as compared to a non-cancer subject is indicative of lung cancer, cytidines being those defined in any one of claims 3 to 8 and cytidine(s) in the sequence of SEQ ID NO: 321.

10. The method of any one of claims 3 to 5 and claims 8 and 9, wherein said determining is effected in a sample taken from a subject, said sample preferably being a bodily fluid, more preferably plasma, serum or sputum; or a biopsy taken from said cancer; a biopsy taken from a metastasis; or a circulating tumor cell.

11. The method of claim 10, wherein prior to said determining,

(a) cell-free DNA comprised in said sample is enriched or isolated;
(b) cell-free DNA is subjected to digestion with one or more methylation-sensitive restriction enzymes; and/or
(c) cell-free DNA is pre-amplified.

12. The method of any one of claims 3 to 5 and claims 8 to 11, wherein said determining is performed by one or more of the following procedures:

(a) a support-based methylation assay;
(b) amplification, preferably PCR, more preferably conventional or real-time PCR, of DNA digested in accordance with claim 11(b) and optionally pre-amplified in accordance with claim 11(c);
(c) a methylated CpG island recovery assay;
(d) a bisulfite assay or bisulfite sequencing;
(e) a proximity hybridization assay; and
(f) nanopore sequencing.

13. The method of any one of claims 3 to 5 and claims 8 to 10, wherein

(a) cell-free DNA comprised in said sample is isolated;
(b) the isolated DNA obtained in (a) is subjected to digestion with one or more methylation-sensitive restriction enzymes;
(c) the digested DNA obtained in (b) is pre-amplified;
(d) the pre-amplified DNA obtained in (c) is amplified.

14. A kit comprising or consisting of the following set of primers:

(1) nucleic acids comprising or consisting of the sequences of SEQ ID NO: 229 and 230 or sequences having at least 80% sequence identity thereto.

15. The kit of claim 14, furthermore comprising one or more of the following:

(a) a manual with instructions for performing the method of any one of claims 3 to 5 and claims 8 to 13;
(b) one or more buffered solutions or reagents for the preparation thereof;
(c) one or more reaction vessels.

**Patentansprüche**

1. Nukleinsäure umfassend oder bestehend aus

(a) der Sequenz
(4) von Position 4 bis Position 84 der SEQ ID Nr.: 1, wobei das Zytidin in mindestens einem in der Sequenz enthaltenen CpG Dinukleotide methyliert ist;
oder
(b) einer Sequenz, die mindestens 80 % Sequenzidentität mit einer Sequenz von (a) aufweist, wobei das Zytidin in mindestens einem in der Sequenz enthaltenen CpG Dinukleotide methyliert ist.

2. Nukleinsäure nach Anspruch 1, wobei mindestens ein, vorzugsweise alle Zytidine an den Positionen
(4) 4, 38, 40, 45 und 83 der SEQ ID Nr.: 1 methyliert ist/sind.

3. Verfahren zur Diagnose von Lungenkrebs oder einer Veranlagung dafür, wobei das Verfahren die Bestimmung des Methylierungszustandes von Zytidin in
(4) mindestens einem CpG Dinukleotid in einer Nukleinsäure umfassend oder bestehend aus Position 4 bis Position 84 der SEQ ID Nr.: 1 umfasst;
wobei die Methylierung des/der Zytidins/e oder erhöhte Methylierung des/der Zytidins/e im Vergleich zu einem Subjekt, das keinen Krebs hat, auf Lungenkrebs oder eine Veranlagung dafür hinweist, und wobei jedes am menschlichen oder tierischen Körper durchgeführte diagnostische Verfahren ausgeschlossen ist.

4. Verfahren zum Verfolgen der Behandlung von Lungenkrebs oder einer Veranlagung dafür, wobei das Verfahren die Bestimmung des Methylierungszustandes von Zytidin umfasst, wobei Zytidin wie in Anspruch 3 definiert ist, wobei keine Methylierung des/der Zytidins/e oder verringerte Methylierung des/der Zytidins/e im Vergleich zu einem Patienten, der Lungenkrebs hat oder einem Individuum, das eine Veranlagung dafür hat, auf eine Besserung des Lungenkrebses oder eine verringerte Veranlagung dafür hinweist, und wobei jedes am menschlichen oder tierischen Körper praktizierte Diagnoseverfahren ausgeschlossen ist.

5. Verfahren nach Anspruch 3 oder 4, wobei das/die Zytidin(e), dessen/deren Methylierungszustand zu bestimmen ist, in Anspruch 2 definiert ist/sind.

6. Nukleinsäure nach Anspruch 1 oder 2 oder Verfahren nach Anspruch 3 oder 4, wobei diese Nukleinsäure eine der SEQ ID Nrn.: 1, 10, 11, 47, 56, 57, 93, 101 und 102 umfasst oder daraus besteht.

7. Nukleinsäure, die aus der SEQ ID Nr.: 137 oder 183 besteht.

8. Verfahren nach einem der Ansprüche 3 bis 5, wobei der Methylierungszustand der Zytidine gemäß

    (1) Punkten (4) und (9);
    (2) Punkten (4) und (2);
    (3) Punkten (4) und (6);
    (5) Punkten (1) und (4);
    (6) Punkten (4) und (5);
    (7) Punkten (4), (2) und (9);
    (8) Punkten (4), (2) und (6);
    (9) Punkten (4), (9) und (10);
    (11) Punkten (4), (9) und (11);
    (12) Punkten (4), (5) und (9);
    (13) Punkten (4), (2) und (10);
    (14) Punkten (1), (4) und (9);
    (15) Punkten (4), (2) und (12);
    (16) Punkten (1), (2) und (4); oder
    (17) Punkten (4), (2) und (5)

zu bestimmen ist, wobei diese Punkte wie folgt definiert sind:
Cytidin in

    (1) mindestens ein CpG Dinukleotid in einer Nukleinsäure, die die Sequenz von Position 7 bis Position 101 der SEQ ID Nr.: 4 umfasst oder daraus besteht;
    (2) mindestens ein CpG Dinukleotid in einer Nukleinsäure, die die Sequenz von Position 11 bis Position 71 der SEQ ID Nr.: 2 umfasst oder daraus besteht;
    (4) definiert ist wie nach einem der Ansprüche 3 bis 7;
    (5) mindestens ein CpG Dinukleotid in einer Nukleinsäure, die die Sequenz von Position 28 bis Position 69 der SEQ ID Nr.: 5 umfasst oder daraus besteht;
    (6) mindestens ein CpG Dinukleotid in einer Nukleinsäure, die die Sequenz von Position 39 bis Position 85 der SEQ ID Nr.: 6 umfasst oder daraus besteht;
    (9) mindestens ein CpG Dinukleotid in einer Nukleinsäure, die die Sequenz von Position 5 bis Position 71 der SEQ ID Nr.: 9 umfasst oder daraus besteht;
    (10) mindestens ein CpG Dinukleotid in einer Nukleinsäure, die die Sequenz von Position 2 bis Position 60 der SEQ ID Nr.: 10 umfasst oder daraus besteht;
    (11) mindestens ein CpG Dinukleotid in einer Nukleinsäure, die die Sequenz von Position 22 bis Position 63 der SEQ ID Nr.: 11 umfasst oder daraus besteht; und
    (12) mindestens ein CpG-Dinukleotid in einer Nukleinsäure, die die Sequenz von Position 45 bis Position 67 der SEQ ID Nr.: 12 umfasst oder daraus besteht.

9. Verfahren nach einem der Ansprüche 3 bis 5 und Anspruch 8, wobei

    (a) der Methylierungszustand keiner weiteren Nukleotide zu bestimmen ist; oder
    (b) das Verfahren ferner das Betimmen des Methylierungszustandes von Zytidin in mindestens einem CpG Dinukleotid in einer Nukleinsäure umfasst, die die Sequenz der SEQ ID Nr.: 321 umfasst oder daraus besteht, wobei Methylierung von Zytidinen oder eine erhöhte Methylierung von Zytidinen im Vergleich zu einem Subjekt, das keinen Krebs hat, auf Lungenkrebs hinweist, wobei Zytidine jene Zytidine sind, die in einem der Ansprüche 3 bis 8 definiert sind und Zytidin(e) in der Sequenz der SEQ ID Nr.: 321.

10. Verfahren nach einem der Ansprüche 3 bis 5 und Ansprüche 8 und 9, wobei das Bestimmen in einer einem Subjekt entnommenen Probe durchgeführt wird, wobei diese Probe vorzugsweise eine Körperflüssigkeit, stärker bevorzugt Plasma, Serum oder Sputum ist; oder eine Biopsie, die dem Krebs entnommen wurde; eine Biopsie, die einer Metastase entnommen wurde; oder eine zirkulierende Tumorzelle.

11. Verfahren nach Anspruch 10, wobei vor dem Bestimmen

(a) in der Probe enthaltene zellfreie DNA angereichert oder isoliert wird;
(b) zellfreie DNA mit einem oder mehreren methylierungssensitiven Restriktionsenzymen verdaut wird; und/oder
(c) zellfreie DNA vor-amplifiziert wird.

12. Verfahren nach einem der Ansprüche 3 bis 5 und Ansprüche 8 bis 11, wobei das Bestimmen mit einem oder mehreren der folgenden Verfahren durchgeführt wird:

(a) einen Träger-basierten Methylierungstest;
(b) Amplifizierung, vorzugsweise PCR, stärker bevorzugt konventionelle oder Echtzeit-PCR, einer DNA, die gemäß Anspruch 11(b) verdaut und gegebenenfalls gemäß Anspruch 11(c) vor-amplifiziert wurde;
(c) einen Wiederfindungstest für methylierte CpG-Inseln;
(d) einen Bisulfittest oder Bisulfitsequenzierung;
(e) einen auf Nähe basierenden Hybridisierungstest; und
(f) Nanoporen-Sequenzierung.

13. Verfahren nach einem der Ansprüche 3 bis 5 und Ansprüche 8 bis 10, wobei

(a) in der Probe enthaltende zellfreie DNA isoliert wird;
(b) die in (a) erhaltene isolierte DNA mit einem oder mehreren methylierungssensitiven Restriktionsenzymen verdaut wird;
(c) die in (b) erhaltene verdaute DNA vor-amplifiziert wird;
(d) die in (c) erhaltene vor-amplifizierte DNA amplifiziert wird.

14. Kit umfassend oder bestehend aus dem folgenden Primer-Set:

(1) Nukleinsäuren umfassend oder bestehend aus den Sequenzen der SEQ ID Nrn.: 229 und 230 oder Sequenzen, die mindestens 80% Sequenzidentität dazu haben.

15. Kit nach Anspruch 14, ferner umfassend eines oder mehrere des Folgenden:

(a) ein Handbuch mit Anleitungen für das Durchführen des Verfahrens nach einem der Ansprüche 3 bis 5 und Ansprüche 8 bis 13;
(b) eine oder mehrere gepufferte Lösungen oder Reagenzien für deren Herstellung;
(c) ein oder mehrere Reaktionsgefäße.

**Revendications**

1. Acide nucléique comprenant ou consistant en

(a) la séquence
(4) de la position 4 à la position 84 de la SEQ ID NO : 1, dans laquelle la cytidine dans au moins un dinucléotide CpG compris dans ladite séquence est méthylée ;
ou
(b) une séquence ayant une identité de séquence d'au moins 80 % avec la séquence de (a) dans laquelle la cytidine dans au moins un dinucléotide CpG compris dans ladite séquence est méthylée.

**2.** Acide nucléique selon la revendication 1, dans lequel au moins une, de préférence la totalité, des cytidines aux positions

(4) 4, 38, 40, 45 et 83 dans la SEQ ID NO : 1 est/sont méthylée(s).

**3.** Méthode de diagnostic d'un cancer du poumon ou d'une prédisposition à celui-ci, ladite méthode comprenant la détermination de l'état de méthylation de cytidines dans

(4) au moins un dinucléotide CpG dans un acide nucléique comprenant ou consistant en la séquence allant de la position 4 à la position 84 de la SEQ ID NO : 1 ;

dans laquelle une méthylation de la ou des cytidine(s) ou une augmentation de la méthylation de la ou des cytidine(s) en comparaison avec un sujet non cancéreux est indicative d'un cancer du poumon ou d'une prédisposition à celui-ci, et dans laquelle une quelconque méthode de diagnostic pratiquée sur le corps humain ou animal est exclue.

**4.** Méthode de surveillance d'un traitement d'un cancer du poumon ou d'une prédisposition à celui-ci, ladite méthode comprenant la détermination de l'état de méthylation de la cytidine, la cytidine étant telle que définie dans la revendication 3, dans laquelle une absence de méthylation de la ou des cytidine(s) ou une réduction de la méthylation de la ou des cytidine(s) en comparaison avec un sujet souffrant d'un cancer du poumon ou d'un sujet prédisposé à celui-ci, respectivement, est indicative d'une amélioration du cancer du poumon ou d'une réduction d'une prédisposition à celui-ci, respectivement, et dans laquelle une quelconque méthode de diagnostic pratiquée sur le corps humain ou animal est exclue.

**5.** Méthode selon la revendication 3 ou 4, dans laquelle la ou les cytidine(s) dont l'état de méthylation doit être déterminé sont celles définies dans la revendication 2.

**6.** Acide nucléique selon la revendication 1 ou 2, ou méthode selon la revendication 3 ou 4, dans lequel ledit acide nucléique comprend ou consiste en l'une quelconque des SEQ ID NO : 1, 10, 11, 47, 56, 57, 93, 101 et 102.

**7.** Acide nucléique consistant en la séquence de la SEQ ID NO : 137 ou 183.

**8.** Méthode selon l'une quelconque des revendications 3 à 5, dans lequel l'état de méthylation des cytidines conformément aux

(1) points (4) et (9) ;
(2) points (4) et (2) ;
(3) points (4) et (6) ;
(5) points (1) et (4) ;
(6) points (4) et (5) ;
(7) points (4), (2) et (9) ;
(8) points (4), (2) et (6) ;
(9) points (4), (9) et (10) ;
(11) points (4), (9) et (11) ;
(12) points (4), (5) et (9) ;
(13) points (4), (2) et (10) ;
(14) points (1), (4) et (9) ;
(15) points (4), (2) et (12) ;
(16) points (1), (2) et (4) ; ou
(17) points (4), (2) et (5)

doit être déterminé, dans laquelle lesdits points sont définis de la manière suivante : cytidine dans

(1) au moins un dinucléotide CpG dans un acide nucléique comprenant ou consistant en la séquence allant de la position 7 à la position 101 de la SEQ ID NO : 4 ;
(2) au moins un dinucléotide CpG dans un acide nucléique comprenant ou consistant en la séquence allant de la position 11 à la position 71 de la SEQ ID NO : 2 ;
(4) tel que défini dans l'une quelconque des revendications 3 à 7 ;
(5) au moins un dinucléotide CpG dans un acide nucléique comprenant ou consistant en la séquence allant de la position 28 à la position 69 de la SEQ ID NO : 5 ;

(6) au moins un dinucléotide CpG dans un acide nucléique comprenant ou consistant en la séquence allant de la position 39 à la position 85 de la SEQ ID NO : 6 ;
(9) au moins un dinucléotide CpG dans un acide nucléique comprenant ou consistant en la séquence allant de la position 5 à la position 71 de la SEQ ID NO : 9 ;
(10) au moins un dinucléotide CpG dans un acide nucléique comprenant ou consistant en la séquence allant de la position 2 à la position 60 de la SEQ ID NO : 10 ;
(11) au moins un dinucléotide CpG dans un acide nucléique comprenant ou consistant en la séquence allant de la position 22 à la position 63 de la SEQ ID NO : 11 ; et
(12) au moins un dinucléotide CpG dans un acide nucléique comprenant ou consistant en la séquence allant de la position 45 à la position 67 de la SEQ ID NO : 12.

9. Méthode selon l'une quelconque des revendications 3 à 5 et 8, dans laquelle

(a) l'état de méthylation d'aucun autre nucléotide ne doit être déterminé ; ou
(b) ladite méthode comprend en outre la détermination de l'état de méthylation de la cytidine dans au moins un dinucléotide CpG dans un acide nucléique comprenant ou consistant en la séquence de la SEQ ID NO : 321, dans laquelle une méthylation de cytidines ou une augmentation de la méthylation de cytidines en comparaison avec un sujet non cancéreux est indicative d'un cancer du poumon, les cytidines étant celles définies dans l'une quelconque des revendications 3 à 8 et la ou les cytidine(s) dans la séquence de la SEQ ID NO : 321.

10. Méthode selon l'une quelconque des revendications 3 à 5 et 8 et 9, dans laquelle ladite détermination est effectuée dans un échantillon pris d'un sujet, ledit échantillon étant de préférence un fluide corporel, mieux encore le plasma, le sérum ou un crachat ; ou une biopsie prise dudit cancer ; une biopsie prise d'une métastase ; ou une cellule tumorale en circulation.

11. Méthode selon la revendication 10, dans laquelle, avant ladite détermination,

(a) l'ADN acellulaire compris dans ledit échantillon est enrichi ou isolé ;
(b) l'ADN acellulaire est soumis à une digestion par une ou plusieurs enzymes de restriction sensibles à une méthylation ; et/ou
(c) l'ADN acellulaire est pré-amplifié.

12. Méthode selon l'une quelconque des revendications 3 à 5 et 8 à 11, dans laquelle ladite détermination est effectuée par une ou plusieurs des procédures suivantes

(a) un dosage de méthylation basé sur un support ;
(b) une amplification, de préférence une PCR, mieux encore une PCR conventionnelle ou en temps réel, d'ADN digéré conformément à la revendication 11(b) et éventuellement préamplifié conformément à la revendication 11(c) ;
(c) un dosage de récupération d'îlot de CpG méthylé ;
(d) un dosage au bisulfite ou un séquençage au bisulfite ;
(e) un dosage d'hybridation de proximité ; et
(f) un séquençage sur nanopores.

13. Méthode selon l'une quelconque des revendications 3 à 5 et 8 à 10, dans laquelle

(a) l'ADN acellulaire compris dans ledit échantillon est isolé ;
(b) l'ADN isolé obtenu en (a) est soumis à une digestion par une ou plusieurs enzymes de restriction sensibles à une méthylation ;
(c) l'ADN digéré obtenu en (b) est préamplifié ;
(d) l'ADN pré-amplifié obtenu en (c) est amplifié.

14. Trousse comprenant ou consistant en les jeux suivants d'amorces :

(1) des acides nucléiques comprenant ou consistant en les séquences des SEQ ID NO : 229 et 230 ou des séquences ayant une identité de séquence d'au moins 80 % avec celles-ci.

15. Trousse selon la revendication 14, comprenant en outre un ou plusieurs des suivants :

(a) un manuel avec des instructions pour mettre en œuvre la méthode de l'une quelconque des revendications 3 à 5 et 8 à 13 ;
(b) une ou plusieurs solutions tamponnées ou des réactifs pour leur préparation ;
(c) un ou plusieurs récipients réactionnels.

Figure 1

A

**Figure 1 continued**

B

cf-DNA levels matched to clinical characteristics

C Separation of healthy persons and cancer patients

D ROC of binominal classifications

Figure 2

**Replicate of patient sample**

p-value =3.375e-10
RHO =0.7131

**Replicate of  patient sample**

p-value =1.733e-17
RHO =0.8687

**Figure 3**

**Separation of cancer and non-cancer**

**ROC Curve & opt. cutpoint**

(0.088, 0.879)

| | cancer |
|---|---|
| Area under Curve | 0.955 |
| Sensitivity | 0.989 |
| Specivity | 0.922 |
| | |
| CutOff | 0.2066 |
| False Positives | 15 |
| False Negatives | 4 |
| Sensitivity | 0.878 |
| Specivity | 0.912 |
| corr.Classification | 90.686 |

**Figure 4**

ROC Curve of prospective samples

**Figure 5**

Samples split into 10 equal parts

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2253714 A
- US 20100273151 A
- US 20110318738 A

### Non-patent literature cited in the description

- **ALBERG et al.** *Chest*, 2013, vol. 143, e1S-29S
- **FAUCI.** Harrison's principles of internal medicine. McGraw-Hill Medical, 2008
- **ESTELLER.** *N Engl J Med*, 2008, vol. 358, 1148-1159
- **BROCK et al.** *N Engl J Med*, 2008, vol. 358, 1118-1128
- **SHINOZAKI et al.** *Clinical cancer research*, 2007, vol. 13, 2068-2074
- **RAUCH et al.** *Cancer Research*, 2006, vol. 66, 7939-7947
- **KALARI et al.** *Oncogene*, 2013, vol. 32, 3559-3568
- **BRAASCH ; COREY.** *Chemistry & Biology*, 2001, vol. 8, 1-7
- **NIELSEN et al.** *Science*, 1991, vol. 254, 1497
- **EGHOLM et al.** *Nature*, 1993, vol. 365, 666
- **DEMIDOV et al.** *Biochem. Pharmacol.*, 1994, vol. 48, 1310-1313
- **WITTUNG et al.** *Nature*, 1994, vol. 368, 561-563
- **RAY ; NORDEN.** *Faseb J.*, 2000, vol. 14, 1041-1060
- **ALTSCHUL et al.** *J. Mol. Biol.*, 1990, vol. 215, 403-410
- **ALTSCHUL ; GISH.** *Methods Enzymol.*, 1996, vol. 266, 460-480
- **PEARSON ; LIPMAN.** *Proc. Natl. Acad. Sci. USA*, 1988, vol. 85, 2444-2448
- **SMITH ; WATERMAN.** *J. Mol. Biol.*, 1981, vol. 147, 195-197
- **HIGGINS et al.** *Nucleic Acids Res.*, 1994, vol. 22, 4673-4680
- *J Mol Biol.*, 1987, vol. 196 (2), 261-82
- **EGGER et al.** *Expert Rev. Mol. Diagn.*, 2012, vol. 12, 75-92
- **WIELSCHER et al.** *BMC Clin Pathol*, 2011, vol. 11, 11
- **FRIEDMANN.** *Annals of Statistics*, 2001, 1189-1232
- **GENTLEMAN.** *Genome biology*, 2004, vol. 5, R80